# EUROPÄISCHE PATENTANMELDUNG

(11) **EP 2 238 964 A2**
(43) Veröffentlichungstag der Anmeldung: **13.10.2010**
(21) Anmeldenummer: 10156740.2
(22) Anmeldetag: 17.03.2010
(51) Int. Cl.: A61K 8/06, A61K 8/37, A61K 8/92, A61Q 19/00

(54) **Stabilisierte kosmetische Zusammensetzungen**

(30) Priorität: 08.04.2009 DE 102009002287
(71) Anmelder: Henkel AG & Co. KGaA, 40589 Düsseldorf (DE)
(72) Erfinder: Bähren, Annika, 41363, Jüchen (DE); Waldmann-Laue, Marianne, 40789, Monheim (DE)

(57) **Zusammenfassung**

Kosmetische Mittel, insbesondere Hautpflegemittel, welche ungesättigte oder aromatische Verbindungen aus der Gruppe der natürlichen Öl- oder Fettkomponenten und Duftstoffe enthalten und trotzdem über das gesamte Produktleben hinweg keine Farb- oder Geruchsveränderungen zeigen, enthalten in einem geeigneten Träger mindestens eine Substanz der allgemeinen Struktur (I) 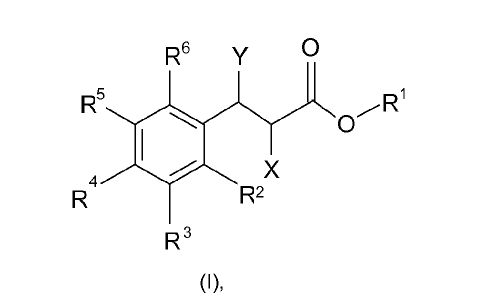 worin
R¹ für ein H-Atom oder eine verzweigte oder lineare C₁-C₃₀-Alkylgruppe oder eine verzweigte oder lineare C₁-C₃₀-Hydroxyalkylgruppe,
R², R⁴ und R⁶ jeweils für ein H-Atom oder eine OH-Gruppe stehen, wobei mindestens einer der Reste R², R⁴ oder R⁶ eine OH-Gruppe ist,
R³ und R⁵ unabhängig voneinander für eine lineare oder verzweigte C₁-C₄-Alkoxygruppe oder eine lineare oder verzweigte C₁-C₆-Alkylgruppe,
X für eine Gruppe -C(=O)OR¹,
und Y für ein H-Atom oder einen gegebenenfalls substituierten Phenylrest Ar stehen, sowie mindestens eine ungesättigte oder aromatische Verbindung, ausgewählt aus natürlichen Öl- oder Fettkomponenten und Duftstoffen, insbesondere Terpenen, die funktionelle Substituenten aufweisen können, gegebenenfalls substituierten Benzylalkoholen und Phenylalkoholen und aldehydischen Duftstoffen.

## Beschreibung

Die vorliegende Erfindung betrifft kosmetische Zusammensetzungen, die mindestens eine ungesättigte oder aromatische Verbindung, ausgewählt aus natürlichen Öl- oder Fettkomponenten und Duftstoffen enthalten.

Der Verbraucher fordert von einem kosmetischen Produkt, dass es neben einer hervorragenden Wirkung im Rahmen seines Hauptanwendungszwecks zusätzlich über den Zeitraum der Anwendung hinweg stabil und in seinen Eigenschaften unverändert bleibt. Zum Schutz der Produkte vor UV-Licht und Einwirkungen des Sauerstoffes der Umgebungsluft werden daher seit langem UV-Filter wie Benzophenone oder Antioxidantien wie Tocopherole eingesetzt.

Leider können die bislang bekannten Substanzen das Bedürfnis nach Schutz vor Veränderung durch Licht und/oder Sauerstoff nicht in allen Kosmetika ausreichend erfüllen. So sind insbesondere ungesättigte oder aromatische Verbindungen aus der Gruppe der natürlichen Öl- oder Fettkomponenten und Duftstoffe durch herkömmliche UV-Filter und Antioxidantien nicht ausreichend gegen Zersetzung zu schützen. Hinzu kommt, dass gerade bei dieser Substanzklasse bereits geringste Veränderungen des kosmetischen Produktes durch den Verbraucher sehr gut wahrnehmbar sind, da übelriechende Produkte entstehen, die eine sehr geringe Wahrnehmungsschwelle besitzen.

Insbesondere bei kosmetischen Hautpflegeprodukten, in denen die genannten Verbindungen oft als kosmetische Ölkörper und Duftstoffe eingesetzt werden, können die herkömmlichen Antioxidantien die Entstehung ranziger Gerüche oder von Farbverschiebungen nicht über das gesamte Produktleben verhindern. Bestimmte Antioxidationsmittel besitzen zudem das Problem, dass ihre starke Eigenfärbung den Einsatz in Hautpflegeprodukten, die oftmals eine reinweiße Färbung aufweisen sollen, verhindert.

Der vorliegenden Erfindung lag die Aufgabe zugrunde, kosmetische Mittel, insbesondere Hautpflegemittel, bereitzustellen, welche ungesättigte oder aromatische Verbindungen aus der Gruppe der natürlichen Öl- oder Fettkomponenten und Duftstoffe enthalten und trotzdem über das gesamte Produktleben hinweg keine Farb- oder Geruchsveränderungen zeigen.

Es wurde nun überraschenderweise gefunden, dass die Aufgabe in hervorragendem Maße durch die Verwendung von Substanzen in den kosmetischen Zubereitungen gelöst wird.

Ein erster Gegenstand der vorliegenden Erfindung ist daher eine kosmetische Zusammensetzung, enthaltend in einem geeigneten Träger
a) mindestens eine Substanz der allgemeinen Struktur (I) 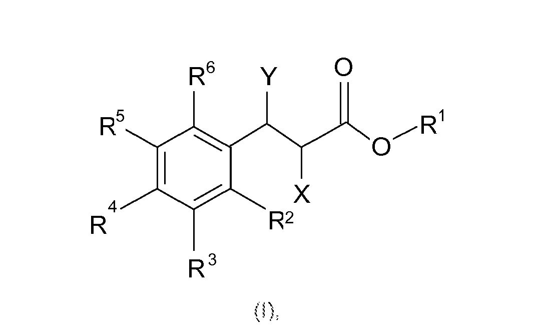 worin
   R¹ für ein H-Atom oder eine verzweigte oder lineare C₁-C₃₀-Alkylgruppe oder eine verzweigte oder lineare C₁-C₃₀-Hydroxyalkylgruppe,
   R², R⁴ und R⁶ jeweils für ein H-Atom oder eine OH-Gruppe stehen, wobei mindestens einer der Reste R², R⁴ oder R⁶ eine OH-Gruppe ist,
   R³ und R⁵ unabhängig voneinander für eine lineare oder verzweigte C₁-C₄-Alkoxygruppe oder eine lineare oder verzweigte C₁-C₆-Alkylgruppe,
   X für eine Gruppe -C(=O)OR¹,
   und Y für ein H-Atom oder einen gegebenenfalls substituierten Phenylrest Ar stehen,
b) mindestens eine ungesättigte oder aromatische Verbindung, ausgewählt aus natürlichen Öl- oder Fettkomponenten und Duftstoffen, insbesondere Terpenen, die funktionelle Substituenten aufweisen können, gegebenenfalls substituierten Benzylalkoholen und Phenylalkoholen und aldehydischen Duftstoffen.

Kosmetische Zusammensetzungen im Sinne der vorliegenden Anmeldung sind Zubereitungen aus Stoffen, die dazu bestimmt sind, äußerlich am Menschen oder in seiner Mundhöhle zur Reinigung, Pflege oder zur Beeinflussung des Aussehens oder des Körpergeruchs oder zur Vermittlung von Geruchseindrücken angewendet zu werden, es sei denn, dass sie überwiegend dazu bestimmt sind, Krankheiten, Leiden, Körperschäden oder krankhafte Beschwerden zu lindern oder zu beseitigen. Je nach Anwendungsgebiet unterscheidet man daher ein breite Palette kosmetischer Mittel, beispielsweise zur Hautpflege (Badepräparate, Hautwasch- und -reinigungsmittel, Hautpflegemittel, Augenkosmetika, Lippenpflegemittel, Nagelpflegemittel, Intimpflegemittel, Fußpflegemittel), solche mit spezieller Wirkung (Lichtschutzmittel, Hautbräunungsmittel, Depigmentierungsmittel, Desodorantien, Antihidrotika, Haarentfernungsmittel, Rasiermittel, Duftmittel), solche zur Zahn- und Mundpflege (Zahn- und Mundpflegemittel, Gebisspflegemittel, Prothesenhaftmittel) und solche zur Haarpflege (Haarwaschmittel, Haarpflegemittel, Haarverfestigungsmittel, Haarverformungsmittel, Mittel zur Farbänderung).

Ganz besonders bevorzugte erfindungsgemäße Mittel dienen der Hautpflege; bevorzugte Anwendungsgebiete der Erfindung sind Hautcremes oder -Lotionen.

Die erfindungsgemäßen kosmetischen Zusammensetzungen enthalten mindestens eine ungesättigte oder aromatische Verbindung, ausgewählt aus natürlichen Öl- oder Fettkomponenten und Duftstoffen, insbesondere Terpenen, die funktionelle Substituenten aufweisen können, gegebenenfalls substituierten Benzylalkoholen und Phenylalkoholen und aldehydischen Duftstoffen.

Unter den natürlichen Öl- oder Fettkomponenten sind insbesondere diejenigen Vertreter zu nennen, die hoch ungesättigte Fettsäuren und/oder einen hohen Anteil an ungesättigten Fettsäuren enthalten. Bei diesen treten die eingangs genannten Probleme besonders deutlich auf. Beispiele für diese bisher problematischen Öle sind Sonnenblumenöl, Distelöl, Erdnussöl, Hanföl, Kürbiskernöl, Maisöl, Mohnöl, Nachtkerzenöl, Olivenöl, Rapsöl, Sesamöl, Traubenkernöl, Walnussöl und Weizenkeimöl.

Mit seinem Gehalt an zweifach ungesättigten Fettsäuren von knapp 80 Gew.-% ist Distelöl bislang sehr schlecht in kosmetische Produkte einarbeit- und stabilisierbar gewesen. Im Rahmen der vorliegenden Erfindung gelingt die Einarbeitung ohne Stabilitätsprobleme oder Ranzigwerden, so dass in bevorzugten erfindungsgemäßen Zusammensetzungen die natürliche Öl- oder Fettkomponente ausgewählt ist aus Distelöl, wobei bevorzugte Zusammensetzungen bezogen auf ihr Gewicht mindestens 2 Gew.-% Distelöl enthalten. Besonders bevorzugte erfindungsgemäße Zusammensetzungen enthalten 2,5 - 10 Gew.-%, außerordentlich bevorzugt 3 - 5 Gew.-%, Distelöl, jeweils bezogen auf die gesamte Zusammensetzung.

Eine weitere Stoffklasse aus der Gruppe der ungesättigten und ggf. aromatischen Duftstoffe stellen die Terpene dar. In erfindungsgemäß bevorzugten Zusammensetzungen ist der mindestens eine Duftstoff, ausgewählt aus Terpenen. In ganz bevorzugten erfindungsgemäßen Zusammensetzungen ist das mindestens eine Terpen ausgewählt aus Monoterpenen, Sesquiterpenen, Diterpenen, Sesterterpenen, Triterpenen, Tetraterpenen und Polyterpenen.

Die bislang sehr schlecht in kosmetische Produkte einarbeit- und stabilisierbaren Terpene Limonen und Bisabolol sind im Rahmen der vorliegenden Erfindung problemlos stabilisierbar, so dass in bevorzugten erfindungsgemäßen Zusammensetzungen das mindestens eine Terpen ausgewählt ist aus Bisabolol und Limonen.

Weitere Stoffe aus der Gruppe der ungesättigten und ggf. aromatischen Duftstoffe sind alpha-Isomethylionon, Amylcinnamal, Amylcinnamylalkohol, Anisalkohol, Benzylcinnamat, Cinnamal, Cinnamylalkohol, Citral, Citronellol, Cumarin, Limonen, Eugenol, Farnesol, Geraniol, Hexylcinnamal, Hydroxycitronellal, Isoeugenol, Lilial, Linalool, Lyral (Hydroxylsohexyl-3-cyclohexencarboxaldehyd) und Methyl-2-octinoat. Auch diese Stoffe lassen sich im Rahmen der vorliegenden Erfindung deutlich besser stabilisieren und zeigen im Gegensatz zu Zusammensetzungen aus dem Stand der Technik keine Geruchsverschiebungen über die Produktlebensdauer auch bei Einwirkung von Licht und Sauerstoff.

Bevorzugte erfindungsgemäße Zusammensetzungen sind daher **dadurch gekennzeichnet, dass** der mindestens eine Duftstoff ausgewählt ist aus alpha-Isomethylionon, Amylcinnamal, Amylcinnamylalkohol, Anisalkohol, Benzylcinnamat, Cinnamal, Cinnamylalkohol, Citral, Citronellol, Cumarin, Limonen, Eugenol, Farnesol, Geraniol, Hexylcinnamal, Hydroxycitronellal, Isoeugenol, Lilial, Linalool, Lyral (Hydroxylsohexyl-3-cyclohexencarboxaldehyd) und Methyl-2-octinoat.

Als zweiten wesentlichen Inhaltsstoff enthalten die erfindungsgemäßen Mittel mindestens eine Substanz der allgemeinen Struktur (I) 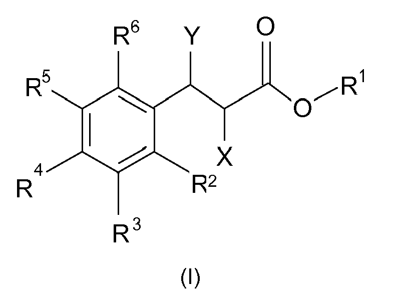 , worin

R¹ für ein H-Atom oder eine verzweigte oder lineare C₁-C₃₀-Alkylgruppe oder eine verzweigte oder lineare C₁-C₃₀-Hydroxyalkylgruppe, R², R⁴ und R⁶ jeweils für ein H-Atom oder eine OH-Gruppe stehen, wobei mindestens einer der Reste R², R⁴ oder R⁶ eine OH-Gruppe ist, R³ und R⁵ unabhängig voneinander für eine lineare oder verzweigte C₁-C₄-Alkoxygruppe oder eine lineare oder verzweigte C₁-C₆-Alkylgruppe, X für eine Gruppe -C(=O)OR¹, und Y für ein H-Atom oder einen gegebenenfalls substituierten Phenylrest Ar stehen.

Besonders bevorzugte Reste R¹ sind Methyl, Ethyl, n-Propyl, Isopropyl, n-Butyl, Isobutyl, tert-Butyl, n-Pentyl, n-Hexyl, n-Heptyl, n-Octyl, Isooctyl, Ethylhexyl, n-Nonyl, n-Decyl, n-Dodecyl, n-Tetradecyl, n-Hexadecyl und n-Octadecyl. In bevorzugten Verbindungen der Formel (I) stehen R² und R⁶ jeweils für ein H-Atom, und R⁴ ist eine OH-Gruppe. Bevorzugte Reste R³ und R⁵ sind unabhängig voneinander Methoxy, Ethoxy, n-Propoxy, Isopropoxy, n-Butyloxy, Isobutyloxy, tert-Butyloxy, Methyl, Ethyl, n-Propyl, Isopropyl, n-Butyl, Isobutyl, tert-Butyl, n-Pentyl, n-Hexyl.

Besonders bevorzugte Substanzen der allgemeinen Struktur (I) lassen sich durch die Formeln (I-a) bis (I-t) beschreiben, in denen die Reste R¹ jeweils für Methyl, Ethyl, n-Propyl, Isopropyl, n-Butyl, Isobutyl, tert-Butyl, n-Pentyl, n-Hexyl, n-Heptyl, n-Octyl, Isooctyl, Ethylhexyl, n-Nonyl, n-Decyl, n-Dodecyl, n-Tetradecyl, n-Hexadecyl und n-Octadecyl stehen. Erfindungsgemäße Mittel, die diese Substanzen enthalten, sind besonders bevorzugt.

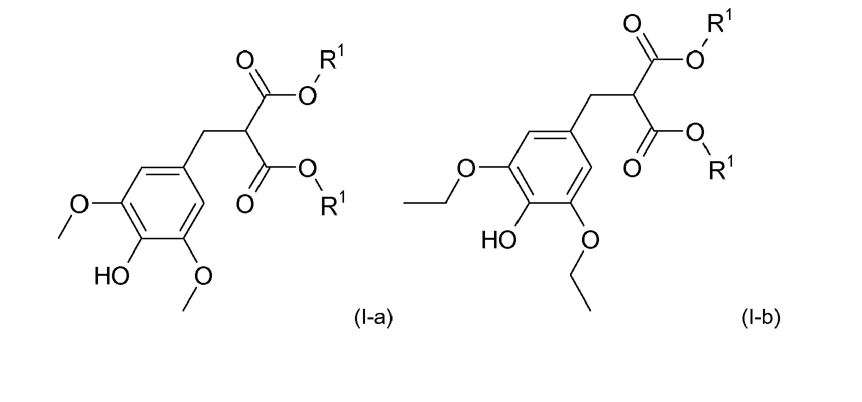

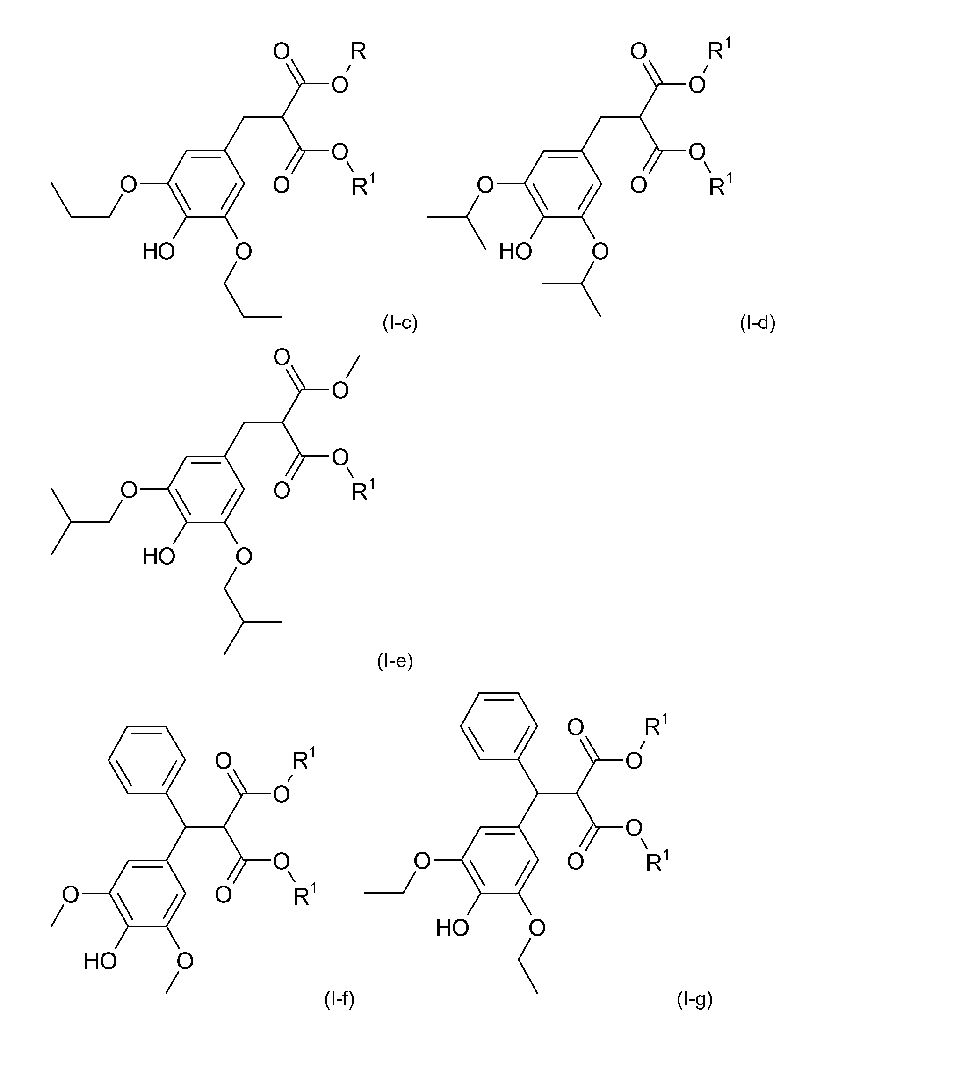

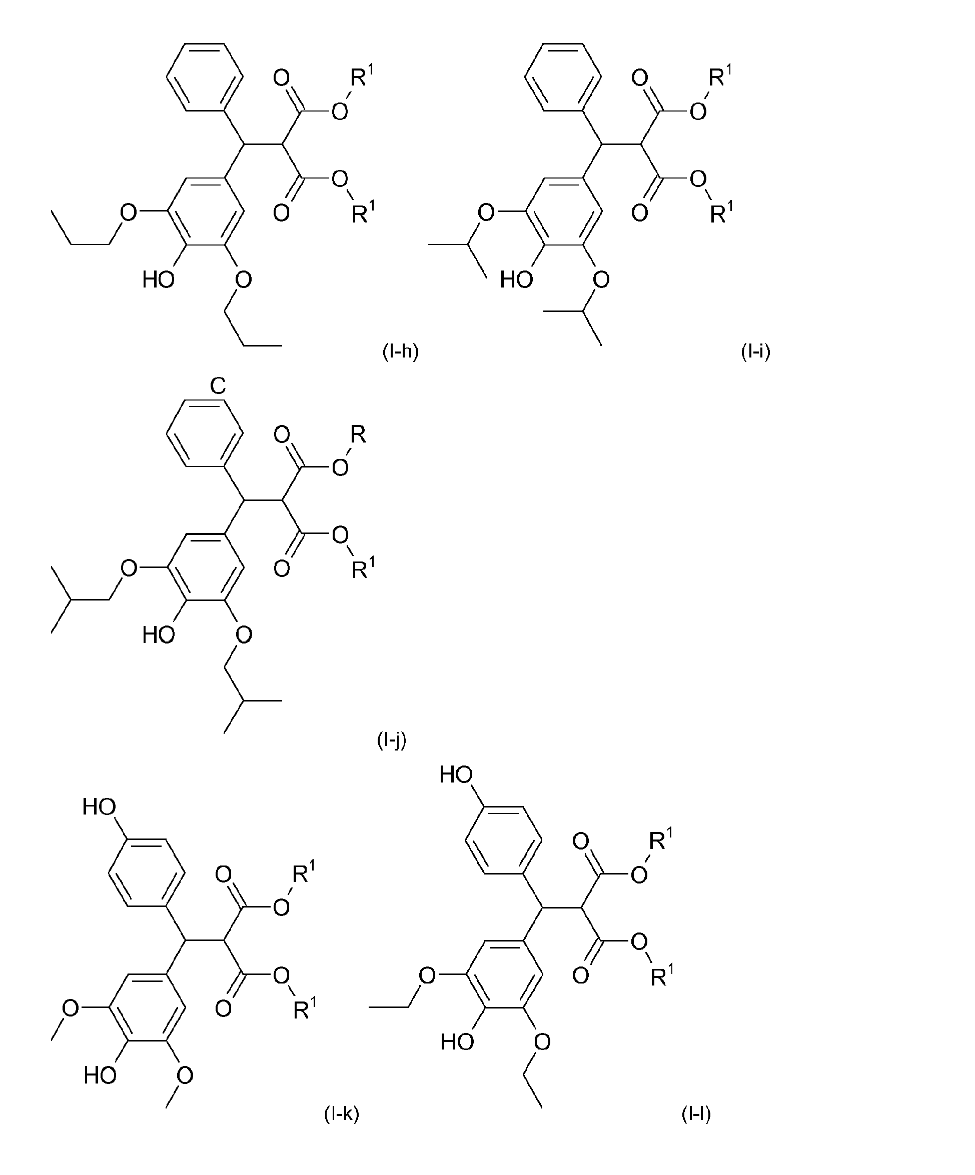

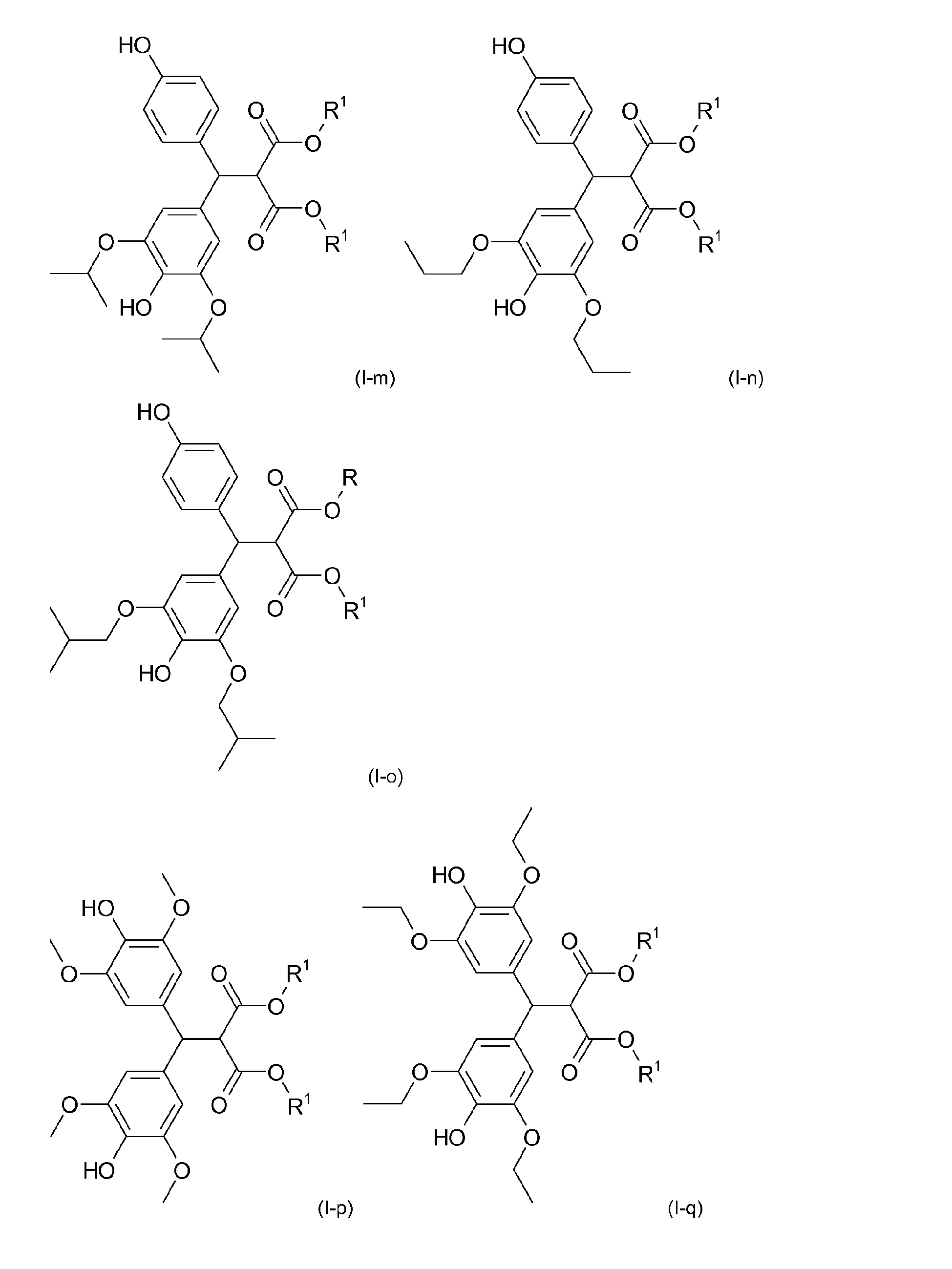

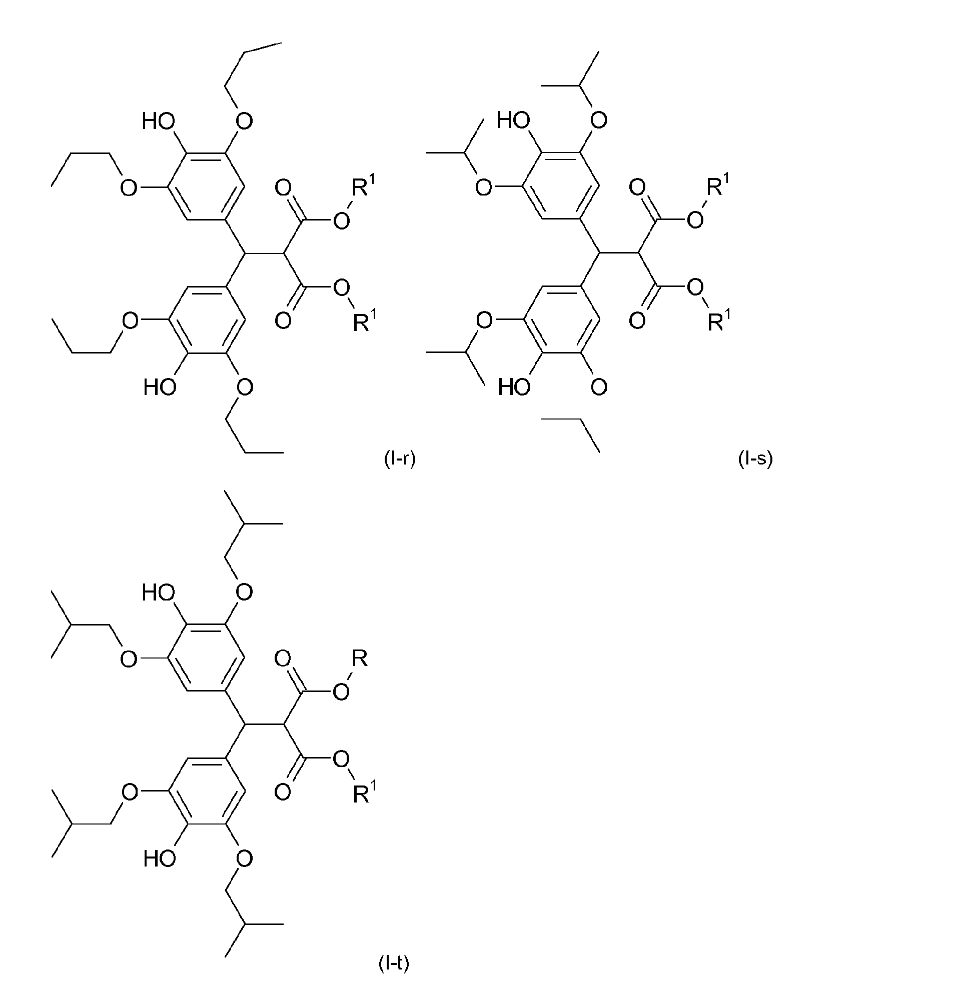

Einige Stoffe der allgemeinen Formel (I) sind besonders bevorzugt. Erfindungsgemäß insbesondere bevorzugte Zusammensetzungen sind **dadurch gekennzeichnet, dass** die Komponente a) ausgewählt ist aus 4-Methoxy-benzyl-malonsäure-di-2-ethyl-hexylester, 4-Hydroxy-3,5-dimethoxy-benzyl-malonsäure-diethylester, 4-Hydroxy-benzyl-malonsäure-di-2-ethyl-hexylester, 3-Hydroxybenzyl-malonsäure-di-2-ethyl-hexylester, 2-Hydroxy-benzyl-malonsäure-di-2-ethyl-hexylester, 3,4,5-Trimethoxy-benzyl-malonsäure-di-2-ethyl-hexylester, 2,4,5-Trimethoxy-benzyl-malonsäure-di-2-ethyl-hexylester, 2,3,4-Trimethoxy-benzyl-malonsäure-di-2-ethyl-hexylester, 2,3, 5-Trimethoxy-benzyl-malonsäure-di-2-ethyl-hexylester, 2,3,6-Trimethoxy-benzyl-malonsäure-di-2-ethyl-hexylester, 2,4,6-Trimethoxy-benzyl-malonsäure-di-2-ethyl-hexylester, 2,4-Dimethoxy-benzyl-malonsäure-di-2-ethyl-hexylester, 2,3-Dimethoxy-benzyl-malonsäure-di-2-ethyl-hexylester, 2,5-Dimethoxy-benzyl-malonsäure-di-2-ethyl-hexylester, 3,4-Dimethoxy-benzyl-malonsäure-di-2-ethyl-hexylester, 3,5-Dimethoxy-benzyl-malonsäure-di-2-ethyl-hexylester, 4-Hydroxy-3-methoxy-benzyl-malonsäure-di-2-ethyl-hexylester, 34,5-Trihydroxy-benzyl-malonsäure-di-2-ethyl-hexylester, 2,4,5-Trihydroxy-benzyl-malonsäure-di-2-ethyl-hexylester, 2,3,4-Trihydroxy-benzyl-malonsäure-di-2-ethyl-hexylester, 2,3,4-Trihydroxy-phenyl-propionsäure-2-ethyl-hexylester, 2,4-Dihydroxy-benzyl-malonsäure-di-2-ethyl-hexylester, 2,3-Dihydroxy-benzyl-malonsäure-di-2-ethyl-hexylester, 2,5-Dihydroxy-benzyl-malonsäure-di-2-ethy-hexytester, 3,4-Dihydroxy-benzyl-malonsäure-di-2-ethyl-hexylester, 3,5-Dihydroxy-benzyl-malonsäure-di-2-ethyl-hexylester, 3-Hydroxy-4-methoxy-benzyl-malonsäure-di-2-ethyl-hexylester, 4-Methoxy-benzyl-malonsäure-diethylester, 3,4,5-Trimethoxy-benzyl-malonsäure-diethylester, 2,4,5-Trimethoxy-benzyl-malonsäure-diethylester, 2,3,4-Trimethoxy-benzyl-matonsäure-diethylester, 2,3,5-Trimethoxy-benzyl-malonsäure-diethylester, 2,3,6-Trimethoxy-benzyl-malonsäure-diethylester, 2,4,6-Trimethoxy-benzyl-malonsäure-diethyester, 2,4-Dimethoxy-benzyl-malonsäure-diethylester, 2,3-Dimethoxy-benzyl-malonsäure-diethylester, 2,5-Dimethoxy-benzyl-malonsäure-diethylester, 3,4-Dimethoxy-benzyl-malonsäure-diethylester, 3,5-Dimethoxy-benzyl-malonsäure-diethylester, 4-Hydroxy-3-methoxy-benzyt-malonsäure-diethylester, 3,4,5-Trihydroxy-benzyl-malonsäure-diethylester, 2,4,5-Trihydroxy-benzyl-malonsäure-diethylester, 2,3,4-Trihyd roxy-benzyl-matonsäure-diethylester, 2,4-Dihydroxy-benzyl-malonsäure-diethylester, 2,3-Dihydroxy-benzyl-malonsäure-diethylester, 2,5-Dihydroxy-benzyl-matonsäure-thethylester, 3,4-Dihydroxy-benzyl-malonsäure-diethylester, 3,5-Dihydroxy-benzyl-malonsäure-diethylester, 3,5-Dihydroxy-phenyl-propionsäure-ethylester, 3-Hydroxy-4-methoxy-benzyl-malonsäure-diethylester, 4-Methoxy-benzyl-malonsäure-diphenethylester, 3,4,5-Trimethoxy-benzyl-malonsäure-diphenethylester, 2,4,5-Trimethoxy-benzyl-malonsäure-diphenethylester, 2,3,4-Trimethoxy-benzyl-malonsäure-diphenethylester, 2,3,5-Trimethoxy-benzyl-malonsäure-diphenethylester, 2,3,6-Trimethoxy-benzyl-malonsäure-diphenethylester, 2,4,6-Trimethoxy-benzyl-malonsäure-diphenethylester, 2,4-Dimethoxy-benzyl-malonsäure-diphenethylester, 2,3-Dimethoxy-benzyl-malonsäure-diphenethylester, 2,5-Dimethoxy-benzyl-malonsäure-diphenethylester, 3,4-Dimethoxy-benzyl-malonsäure-diphenethylester, 3,5-Dimethoxy-benzyl-malonsäure-diphenethylester, 4-Hydroxy-3-methoxy-benzyl-malonsäure-diphenethylester, 3,4,5-Trihydroxy-benzyl-malonsäure-diphenethylester, 2,4,5-Trihydroxy-benzyl-malonsäure-diphenethylester, 2,3,4-Trihydroxy-benzyl-malonsäure-diphenethylester, 2,4-Dihydroxy-benzyl-malonsäure-diphenethylester, 2,3-Dihydroxy-benzyl-malonsäure-diphenethylester, 2,5-Dihydroxy-benzyl-malonsäure-diphenethylester, 3,4-Dihydroxy-benzy-malonsaure-diphenethylester, 3,5-Dihydroxy-benzyl-malonsäure-diphenethylester, 3-Hydroxy-4-methoxy-benzyl-malonsäure-diphenethylester, sowie deren Mischungen.

Erfindungsgemäß ganz besonders bevorzugte Zusammensetzungen sind **dadurch gekennzeichnet, dass** die Komponente a) ausgewählt ist aus [(4-Hydroxy-3,5-dimethoxybenzyl]-malonsäure-bis-(2-ethylhexyl)ester (Formel I-u).

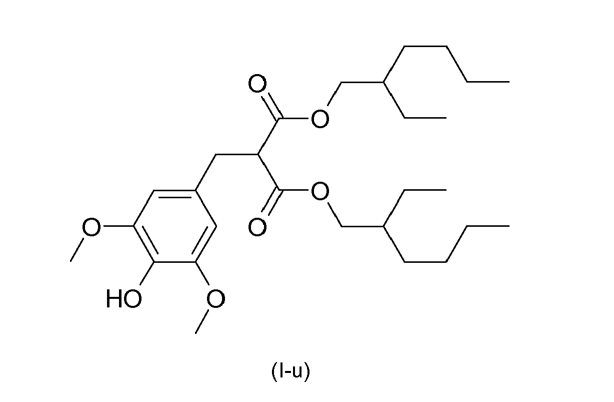

Bevorzugte erfindungsgemäße Zusammensetzungen enthalten die Verbindung(en) der Formel (I) in Mengen von 0,001 bis 10 Gew.-%, jeweils bezogen auf die gesamten Zusammensetzungen. Weiter bevorzugt sind Einsatzmengen von 0,005 bis 7,5 Gew.-%, vorzugsweise von 0,01 bis 5 Gew.-%, weiter bevorzugt von 0,05 bis 2,5 Gew.-%, noch weiter bevorzugt von 0,1 bis 1 Gew.-% und insbesondere von 0,2 bis 0,6 Gew.-%, jeweils bezogen auf das Gesamtgewicht der Zusammensetzung.

Ganz besonders bevorzugte erfindungsgemäße Mittel enthalten - bezogen auf ihr Gewicht - 0,05 bis 5 Gew.-%, vorzugsweise 0,075 bis 4 Gew.-%, weiter bevorzugt 0,1 bis 3 Gew.-%, noch weiter bevorzugt 0,15 bis 2 Gew.-%, besonders bevorzugt 0,2 bis 1 Gew.-% und insbesondere 0,25 bis 0,6 Gew.-% [(4-Hydroxy-3,5-dimethoxybenzyl]-malonsäure-bis-(2-ethylhexyl)ester.

Die erfindungsgemäßen Zusammensetzungen können darüber hinaus weitere UV-Filter und/oder Antioxidantien enthalten. Besonders bevorzugt ist der zusätzliche Einsatz von Tocopherolen, die in ihrer Wirkung durch die Komponente a) synergistisch verstärkt werden. Bevorzugte erfindungsgemäße Zusammensetzungen sind **dadurch gekennzeichnet, dass** weiterhin Tocopherol oder ein Tocopherylester enthalten ist.

Ganz besonders bevorzugte erfindungsgemäße Mittel enthalten - bezogen auf ihr Gewicht - 0,01 bis 2,5 Gew.-%, vorzugsweise 0,05 bis 2 Gew.-%, weiter bevorzugt 0,1 bis 1,5 Gew.-%, noch weiter bevorzugt 0,15 bis 1 Gew.-%, besonders bevorzugt 0,2 bis 0,7 Gew.-% und insbesondere 0,25 bis 0,6 Gew.-% alpha-Tocopherylacetat.

Als günstiges, aber dennoch fakultativ zu verwendendes zusätzliches Antioxidans kann weiterhin Dimethylmethoxy Chromanol, erhältlich unter dem Handelsnamen Lipochroman-6 von Lipotec, eingesetzt werden.

Weiterhin sind erfindungsgemäße Zusammensetzungen bevorzugt, die **dadurch gekennzeichnet sind, dass** Catechine, Proanthocyanidine, Anthocyanidine und Gallotannine in einer Gesamtmenge von 0 - 0,001 Gew.-% enthalten sind.

Als einen bevorzugten weiteren optionalen Inhaltsstoff enthalten die erfindungsgemäßen Zusammensetzungen Purin und/oder mindestens ein Derivat des Purins. Purin (7*H*-Imidazo[4,5-*d*]pyrimidin) kommt frei in der Natur nicht vor, bildet jedoch den Grundkörper der Purine. Purine ihrerseits sind eine Gruppe wichtiger, in der Natur weit verbreiteter und an menschlichen, tierischen, pflanzlichen und mikrobiellen Stoffwechselvorgängen beteiligter Verbindungen, die sich vom Grundkörper durch Substitution mit OH, NH₂, SH in 2-, 6- und 8-Stellung und/oder mit CH₃ in 1-, 3-, 7-Stellung ableiten. Purin kann beispielsweise aus Aminoacetonitril und Formamid hergestellt werden. Purine und Purinderivate werden oft aus Naturstoffen isoliert, sind aber auch auf vielen Wegen synthetisch zugänglich.

Bevorzugte erfindungsgemäße Zusammensetzungen enthalten Purin und/oder Purinderivat(e) in engeren Mengenbereichen. Hier sind erfindungsgemäß bevorzugte kosmetische Zusammensetzungen **dadurch gekennzeichnet, dass** sie - bezogen auf ihr Gewicht - eine Gesamtmenge von 0,001 - 2,5 Gew.-%, bevorzugt 0,01 - 1 Gew.-%, besonders bevorzugt 0,1 - 0,8 Gew.-% und insbesondere 0,2 - 0,5 Gew.-% Purin(e) und/oder Purinderivat(e) enthalten.

Unter Purin, den Purinen und den Purinderivaten sind einige Vertreter erfindungsgemäß besonders bevorzugt. Erfindungsgemäß bevorzugte kosmetische Zusammensetzungen sind **dadurch gekennzeichnet, dass** sie Purin und/oder Purinderivat(e) der Formel (PUR-I) enthalten, 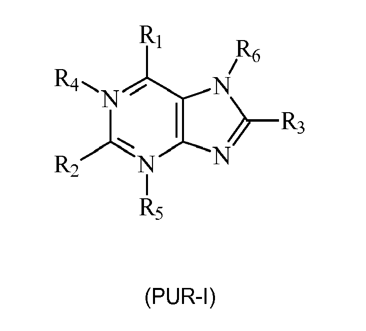 in der die Reste R¹, R² und R³ unabhängig voneinander ausgewählt sind aus -H, - OH, -NH₂ und -SH und die Reste R⁴, R⁵ und R⁶ unabhängig voneinander ausgewählt sind aus -H, einem C₁- bis C₄-Alkylrest, einem C₁- bis C₄-Monohydroxyalkylrest, einem C₂- bis C₄-Polyhydroxyalkylrest, einem (C₁- bis C₄)-Alkoxy-(C₁- bis C₄)-alkylrest, einem C₁- bis C₄-Aminoalkylrest, einem Hydroxy-(C₁- bis C₄)-alkylaminorest, einem C₁- bis C₄-Hydroxyalkoxyrest, einem C₁- bis C₄-Hydroxyalkyl-(C₁-bis C₄)-aminoalkylrest oder einem (Di-C₁- bis C₄-Alkylamino)-(C₁- bis C₄)-alkylrest, einer gegebenenfalls substituierten Arylgruppe, insbesondere einer Phenylgruppe, einer gegebenenfalls substituierten Heteroarylgruppe und einer Aryl-C₁-C₆-alkylgruppe.

Erfindungsgemäß besonders bevorzugte kosmetische Zusammensetzungen sind **dadurch gekennzeichnet, dass** sie Purin und/oder Purinderivat(e) der Formel (PUR-I) enthalten, in der die Reste R¹, R² und R³ unabhängig voneinander ausgewählt sind aus -H, - OH, -NH₂, -SH und die Reste R⁴, R⁵ und R⁶ unabhängig voneinander ausgewählt sind aus -H, -CH₃ und -CH₂-CH₃, wobei folgende Verbindungen außerordentlich bevorzugt sind:
- Purin(R¹ = R² = R³ = R⁴ = R⁵ = R⁶ = H)
- Adenin (R¹ = NH₂, R² = R³ = R⁴ = R⁵ = R⁶ = H)
- Guanin (R'= OH, R = NH₂, R³ = R⁴ = R⁵ = R⁶ = H)
- Harnsäure (R¹ = R² = R³ = OH, R⁴ = R⁵ = R⁶ = H)
- Hypoxanthin (R¹ = OH, R² = R³ = R⁴ = R⁵ = R⁶ = H)
- 6-Purinthiol (R¹ = SH, R² = R³ = R⁴ = R⁵ = R⁶ = H)
- 6-Thioguanin (R¹ = SH, R² = NH₂, R³ = R⁴ = R⁵ = R⁶ = H)
- Xanthin (R¹ = R² = OH, R³ = R⁴ = R⁵ = R⁶ = H)
- Coffein (R¹ = R² = OH, R³ = H, R⁴ = R⁵ = R⁶ = CH₃)
- Theobromin (R¹ = R² = OH, R³ = R⁴ = H, R⁵ = R⁶ = CH₃)
- Theophyllin (R¹ = R² = OH, R³ = H, R⁴ = CH₃, R⁵ = CH₃, R⁶ = H).

Verbindungen der allgemeinen Formel (PUR-I), in denen R¹ und/oder R² einer OH-Gruppe entsprechen, existieren in tautomeren Verbindungen, insbesondere solchen der allgemeinen Formel (PUR-II), ausgehend von (PUR-I) mit R¹ = R² = OH : 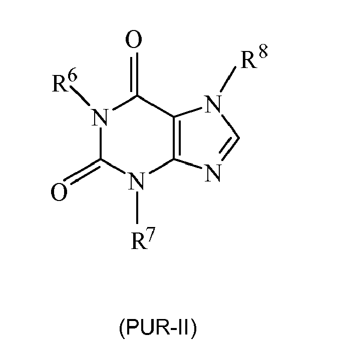 wobei in der Formel (PUR-II) die Reste R⁶, R⁷ und R⁸, die gleich oder verschieden sein können, bevorzugt ausgewählt sind aus einem Wasserstoffatom, einem C₁- bis C₄-Alkylrest, einem C₁- bis C₄-Monohydroxyalkylrest, einem C₂- bis C₄-Polyhydroxyalkylrest, einem (C₁- bis C₄)-Alkoxy-(C₁-bis C₄)-alkylrest, einem C₁- bis C₄-Aminoalkylrest, einem Hydroxy-(C₁- bis C₄)-alkylaminorest, einem C₁- bis C₄-Hydroxyalkoxyrest, einem C₁- bis C₄-Hydroxyalkyl-(C₁-bis C₄)-aminoalkylrest oder einem (Di-C₁- bis C₄-Alkylamino)-(C₁- bis C₄)-alkylrest, einer gegebenenfalls substituierten Arylgruppe, insbesondere einer Phenylgruppe, einer gegebenenfalls substituierten Heteroarylgruppe und einer Aryl-C₁-C₆-alkylgruppe, wobei Coffein (R⁶ = R⁷ = R⁸ = CH₃), Theobromin (R⁶ = H, R⁷ = R⁸ = CH₃) und Theophyllin (R⁶ = R⁷ = CH₃, R⁸ = H) außerordentlich bevorzugt sind.

Je nach gewünschtem Anwendungszweck der kosmetischen Zusammensetzung kann dabei die Art und Menge des Purinderivates variieren. In haarkosmetischen Formulierungen und in Körperpflegemitteln zur Anticellulite-Behandlung der Haut, hat sich insbesondere Coffein bewährt, das beispielsweise in Shampoos vorzugsweise in Mengen von 0,005 bis 0,25 Gew.-%, weiter bevorzugt von 0,01 bis 0,1 Gew.-% und insbesondere von 0,01 bis 0,05 Gew.-% (jeweils bezogen auf das Shampoo) und in Anticellulite-Mitteln, insbesondere Cremes, Gelen und Lotionen, vorzugsweise in Mengen von 0,01 -2 Gew.-%, weiter bevorzugt 0,1 - 1,5 Gew.-% und besonders bevorzugt 0,2 - 0,8 Gew.-%, jeweils bezogen auf das Mittel, eingesetzt werden kann.

Weitere bevorzugte erfindungsgemäße Zusammensetzungen sind **dadurch gekennzeichnet, dass** sie zusätzlich zu der Wirkstoffkombination a) und b) als weiteren Inhaltsstoff mindestens eine natürliche Betainverbindung enthalten. Erfindungsgemäß eingesetzte natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N+ -CH₂-COO⁻) und Carnitin (Me₃N⁺-CH₂-CHOH-CH₂-COO⁻), jeweils mit Me = Methyl.

Bevorzugte erfindungsgemäße Zusammensetzungen sind **dadurch gekennzeichnet, dass** sie mindestens eine natürliche Betainverbindung in einer Gesamtmenge von 0,01 bis 5 Gew.-%, bevorzugt 0,1 bis 3 Gew.-%, besonders bevorzugt 0,2 bis 1 Gew.-% und außerordentlich bevorzugt 0,3 - 0,5 Gew.-%, jeweils bezogen auf die gesamte Zusammensetzung, enthalten.

Weitere bevorzugte erfindungsgemäße Zusammensetzungen sind **dadurch gekennzeichnet, dass** sie zusätzlich zu der Wirkstoffkombination a) und b) als weiteren Inhaltsstoff mindestens eine Substanz enthalten, die ausgewählt ist aus Harnstoff und alkyl- oder hydroxyalkylsubstituierten Harnstoffen.

Erfindungsgemäß bevorzugte alkyl- oder hydroxyalkylsubstituierte Harnstoffverbindungen weisen die allgemeine Formel (UREA-I) auf, 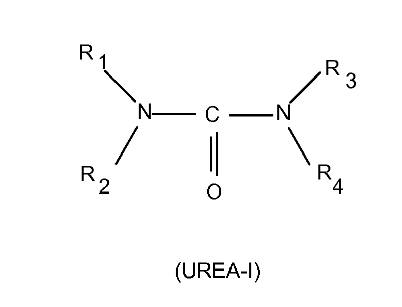 in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ - R₄ eine C₂-C₆-Hydroxyalkyl-Gruppe, die mit 1 bis 5 Hydroxylgruppen oder C₁-C₄-Hydroxyalkyl-Gruppen substituiert ist, darstellt.

Erfindungsgemäß besonders bevorzugte alkyl- oder hydroxyalkylsubstituierte Harnstoffverbindungen der allgemeinen Formel (UREA-I) sind ausgewählt aus Verbindungen, bei denen jeweils mindestens einer der Reste R₁ und R₂ bzw. R₃ und R₄ eine C₂-C₆-Hydroxyalkyl-Gruppe, die mit 1 bis 5 Hydroxylgruppen oder C₁-C₄-Hydroxyalkyl-Gruppen substituiert ist, darstellt. Besonders bevorzugte C₂-C₆-Hydroxyalkyl-Gruppen, die mit 1 bis 5 Hydroxylgruppen oder C₁-C₄-HydroxyalkylGruppen substituiert sind, sind ausgewählt aus 2-Hydroxyethyl-, 3-Hydroxypropyl-, 2-Hydroxypropyl, 2-Hydroxylsopropyl- und 4-Hydroxybutyl-Gruppen, wobei die 2-Hydroxyethyl-Gruppe außerordentlich bevorzugt ist. Ebenfalls außerordentlich bevorzugt ist Bis-N,N'-(2-Hydroxyethyl)-harnstoff. Harnstoff selbst ist ebenfalls besonders bevorzugt. Weiterhin bevorzugt ist es, Mischungen von Harnstoff und alkyl- oder hydroxyalkylsubstituierten Harnstoffverbindungen einzusetzen. Außerordentlich bevorzugt sind Mischungen von Harnstoff und Bis-N,N'-(2-Hydroxyethyl)-harnstoff.

Weitere bevorzugte erfindungsgemäße Zusammensetzungen sind **dadurch gekennzeichnet, dass** sie zusätzlich zu der Wirkstoffkombination a) und b) als weiteren Inhaltsstoff mindestens eine Substanz enthalten, die ausgewählt ist aus Monomeren, Oligomeren und Polymeren von Aminosäuren, N-C₂-C₂₄-Acylaminosäuren und/oder den Estern und/oder den physiologisch verträglichen Salzen dieser Substanzen, sowie Mischungen dieser Wirkstoffe.

Die Monomere der Aminosäuren und/oder der N-C₂-C₂₄-Acylaminosäuren sind bevorzugt ausgewählt aus Alanin, Arginin, Asparagin, Asparaginsäure, Canavanin, Citrullin, Cystein, Cystin, Dipalmitoylhydroxyprolin, Desmosin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, N-Acetyl-L-cystein, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Lysin, Serin, Zink- und Natriumpyroglutamat und Natriumlauroylglutamat.

Der C₂ - C₂₄-Acylrest, mit dem die genannten Aminosäuren an der Aminogruppe derivatisiert sind, ist bevorzugt ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten.

Mit den vorgenannten C₂ - C₂₄-Acylresten können die Aminosäuren, die eine OH-Gruppe tragen, auch an dieser OH-Gruppe verestert sein. Ein erfindungsgemäß bevorzugtes Beispiel hierfür ist Hydroxyprolin, das mit zwei, bevorzugt linearen, C₂-C₂₂-Fettsäureresten N-acyliert und verestert ist, besonders bevorzugt Dipalmitoylhydroxyprolin, das z. B. unter der Bezeichnung Sepilift DPHP von der Firma Seppic erhältlich ist.

Die physiologisch verträglichen Salze der erfindungsgemäß bevorzugten Aminosäuren oder Aminosäurederivate sind bevorzugt ausgewählt aus den Ammonium-, Alkalimetall-, Magnesium-, Calcium-, Aluminium-, Zink- und Mangan-Salzen. Besonders bevorzugt sind die Natrium-, Kalium-, Magnesium-, Zink- und Mangan-Salze; außerordentlich bevorzugt sind die Natrium-, Kalium-und Magnesiumsalze.

Unter Aminosäureoligomeren werden erfindungsgemäß Peptide mit 2 - 30, bevorzugt 2 - 15, Aminosäuren, verstanden. Die Oligomere der Aminosäuren und/oder der N-C₂-C₂₄-Acylaminosäuren sind bevorzugt ausgewählt aus Di-, Tri-, Tetra-, Penta-, Hexa- oder Pentadecapeptiden, die N-acyliert und/ oder verestert sein können. Zahlreiche dieser Aminosäureoligomere stimulieren die Collagensynthese beziehungsweise sind in der Lage, Zellen des Immunsystems, wie Mastzellen und Makrophagen, zu rekrutieren, die dann über die Freisetzung von Wachstumsfaktoren Reparaturprozesse im Gewebe, z.B. die Collagensynthese, induzieren, beziehungsweise sind in der Lage, an die Sequenz Arg-Phe-Lys in Thrombospondin I (TSP-1) zu binden und damit aktives TGF-β (*tissue growth factor*), der die Synthese von Collagen in dermalen Fibroblasten induziert, freizusetzen. Derartige Aminosäureoligomere werden im Stand der Technik als Wirkstoffe gegen die Hautalterung verwendet.

Erfindungsgemäß bevorzugte, gegebenenfalls N-acylierte und/oder veresterte Dipeptide sind Acetyl-Citrullyl-Arginin (z. B. Exsy-Algine von Exsymol mit der INCI-Bezeichnung Acetyl Citrull Amido Arginine), Tyr-Arg (Dipeptide-1), Val-Trp (Dipeptide-2), Asn-Phe, Asp-Phe, N-Palmitoyl-β-Ala-His, N-Acetyl-Tyr-Arg-hexyldecylester (z. B. Calmosensine von Sederma), Carnosin (β-Ala-His) und N-Palmitoyl-Pro-Arg. Erfindungsgemäß bevorzugte, gegebenenfalls N-acylierte und/oder veresterte Tripeptide sind Gly-His-Lys, das z. B. unter der Bezeichnung "Omega-CH-Aktivator" von der Firma GfN oder in acylierter Form (N-Palmitoyl-Gly-His-Lys) unter der Bezeichnung Biopeptide CL von Sederma erhältlich ist, aber (in acylierter Form) auch einen Bestandteil des Produktes Matrixyl 3000 von Sederma darstellt. Das Tripeptid Gly-His-Lys kann auch als Kupfersalz (Cu²⁺) eingesetzt werden und ist als solches über ProCyte Corporation zu beziehen. Weiterhin können Analoga von Gly-His-Lys eingesetzt werden, wobei maximal zwei Aminosäuren durch geeignete andere Aminosäuren substituiert sind. Zur Substitution von Gly sind erfindungsgemäß Ala, Leu und Ile geeignet. Die erfindungsgemäß bevorzugten Aminosäuren, die His oder Lys ersetzen können, beinhalten eine Seitenkette mit einem Stickstoffatom, das bei pH 6 überwiegend geladen vorliegt, z. B. Pro, Lys, Arg, His, Desmosin und Isodesmosin. Besonders bevorzugt wird Lys durch Arg, Orn, oder Citrullin ersetzt. Ein weiteres erfindungsgemäß bevorzugtes Tripeptid ist Gly-His-Arg (INCI-Bezeichnung: Tripeptide-3) sowie dessen Derivat N-Myristoyl-Gly-His-Arg, das z. B. unter der Bezeichnung Collasyn 314-GR von Therapeutic Peptide Inc. erhältlich ist; weitere erfindungsgemäß bevorzugte Tripeptide sind ausgewählt aus Lys-Val-Lys, Lys-Val-Dab (Dab = Diaminobuttersäure), Lys-Phe-Lys, Lys-Ile-Lys, Dab-Val-Lys, Lys-Val-Orn, Lys-Val-Dap (Dap = Diaminopropionsäure), Dap-Val-Lys, Palmitoyl-Lys-Val-Lys, z. B. erhältlich von der Firma Pentapharm unter der Bezeichnung SYN^{®}-COLL, Lys-Pro-Val, Tyr-Tyr-Val, Tyr-Val-Tyr, Val-Tyr-Val (Tripeptide-2), Tripeptide-4 (z. B. ATPeptide, zu beziehen über IMPAG), His-Ala-Orn, Gly-Asp-Ser, N-Elaidoyl-Lys-Phe-Lys und N-Acetyl-Arg-Lys-Arg-NH₂.

Erfindungsgemäß bevorzugte, gegebenenfalls N-acylierte und/oder veresterte Tetrapeptide sind ausgewählt aus Rigin und Rigin-basierten Tetrapeptiden sowie ALAMCAT-Tetrapeptiden. Rigin weist die Sequenz Gly-Gln-Pro-Arg auf. Rigin-basierte Tetrapeptide umfassen die Rigin-Analoga und Rigin-Derivate, insbesondere das erfindungsgemäß besonders bevorzugte N-Palmitoyl-Gly-Gln-Pro-Arg, das z. B. unter der Bezeichnung Eyeliss von Sederma erhältlich ist, aber auch einen Bestandteil des Produktes Matrixyl 3000 von Sederma darstellt. Zu den Rigin-Analoga zählen solche, bei denen die vier Aminosäuren umarrangiert sind und/oder bei denen gegenüber Rigin maximal zwei Aminosäuren substituiert sind, z. B. die Sequenz Ala-Gln-Thr-Arg. Bevorzugt hat mindestens eine der Aminosäuren der Sequenz ein Pro oder Arg und besonders bevorzugt beinhaltet das Tetrapeptid sowohl Pro als auch Arg, wobei ihre Reihenfolge und Position variieren können. Die substituierenden Aminosäuren können aus jeder Aminosäure, die im Folgenden definiert ist, ausgewählt werden. Besonders bevorzugte Rigin-basierte Tetrapetide umfassen: Xaa-Xbb-Arg-Xcc, Xaa-Xbb-Xcc-Pro, Xaa-Xbb-Pro-Arg, Xaa-Xbb-Pro-Xcc, Xaa-Xbb-Xcc-Arg, wobei Xaa, Xbb und Xcc gleiche oder voneinander verschiedene Aminosäuren sein können und wobei Xaa ausgewählt ist aus Gly und den Aminosäuren, die Gly substituieren können, Xbb ausgewählt ist aus Gln und den Aminosäuren, die Gln substituieren können, Xcc ausgewählt ist aus Pro oder Arg und den Aminosäuren, die Pro und Arg substituieren können.

Die bevorzugten Aminosäuren, die Gly ersetzen können, beinhalten eine aliphatische Seitenkette, z. B. β-Ala, Ala, Val, Leu, Pro, Sarcosin (Sar) und Isoleucin (Ile).

Die bevorzugten Aminosäuren, die Gln ersetzen können, beinhalten eine Seitenkette mit einer Aminogruppe, die bei neutralem pH (pH 6-7) überwiegend ungeladen vorliegt, z.B. Asn, Lys, Orn, 5-Hydroxyprolin, Citrullin und Canavanin.

Die bevorzugten Aminosäuren, die Arg ersetzen können, beinhalten eine Seitenkette mit einem Stickstoffatom, das bei pH 6 überwiegend geladen vorliegt, z.B. Pro, Lys, His, Desmosin und Isodesmosin.

Als Rigin-Analoga sind erfindungsgemäß Gly-Gln-Arg-Pro und Val-Val-Arg-Pro bevorzugt.

ALAMCAT-Tetrapeptide sind Tetrapeptide, die mindestens eine Aminosäure mit einer aliphatischen Seitenkette enthalten, z. B. β-Ala, Ala, Val, Leu, Pro, Sarcosin (Sar) und Isoleucin (Ile). Weiterhin beinhalten ALAMCAT-Tetrapeptide mindestens eine Aminosäure mit einer Seitenkette mit einer Aminogruppe, die bei neutralem pH (pH 6-7) überwiegend ungeladen vorliegt, z.B. Gln, Asn, Lys, Orn, 5-Hydroxyprolin, Citrullin und Canavanin. Weiterhin beinhalten ALAMCAT-Tetrapeptide mindestens eine Aminosäure mit einer Seitenkette mit einem Stickstoffatom, das bei pH 6 überwiegend geladen vorliegt, z. B. Arg, Pro, Lys, His, Desmosin und Isodesmosin. Als vierte Aminosäure können ALAMCAT-Tetrapeptide jede beliebige Aminosäure enthalten; bevorzugt ist jedoch auch die vierte Aminosäure aus den drei vorstehend genannten Gruppen ausgewählt.

Erfindungsgemäß bevorzugte, gegebenenfalls N-acylierte und/oder veresterte Pentapeptide sind ausgewählt aus Lys-Thr-Thr-Lys-Ser und seinen N-acylierten Derivaten, besonders bevorzugt N-Palmitoyl-Lys-Thr-Thr-Lys-Ser, das unter der Bezeichnung Matrixyl von der Firma Sederma erhältlich ist, weiterhin N-Palmitoyl-Tyr-Gly-Gly-Phe-Met, Val-Val-Arg-Pro-Pro, N-Palmitoyl-Tyr-Gly-Gly-Phe-Leu, Gly-Pro-Phe-Pro-Leu und N-Benzyloxycarbonyl-Gly-Pro-Phe-Pro-Leu (die beiden letztgenannten stellen Serinproteinase-Inhibitoren zur Inhibition der Desquamation dar). Erfindungsgemäß bevorzugte, gegebenenfalls N-acylierte und/oder veresterte Hexapeptide sind Val-Gly-Val-Ala-Pro-Gly und seine N-acylierten Derivate, besonders bevorzugt N-Palmitoyl-Val-Gly-Val-Ala-Pro-Gly, das unter der Bezeichnung Biopeptide EL von der Firma Sederma erhältlich ist, weiterhin Glu-Glu-Met-Gln-Arg-Arg, weiterhin Acetyl-Hexapeptide-3 (Argireline von Lipotec), Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience) und Hexapeptide-10 (z. B. Collaxyl von Vincience oder Seriseline von Lipotec), Ala-Arg-His-Leu-Phe-Trp (Hexapeptide-1), Acetyl Hexapeptide-1 (z. B. Modulene von Vincience), Acetyl Glutamyl Hexapeptide-1 (z. B. SNAP-7 von Centerchem), Hexapeptide-2 (z. B. Melanostatine-DM von Vincience), Ala-Asp-Leu-Lys-Pro-Thr (Hexapeptide-3, z. B. Peptide 02 von Vincience), Val-Val-Arg-Pro-Pro-Pro, Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Ala-Arg-His-Methylnorleucin-Homophenylalanin-Trp (Hexapeptide-7), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience), Hexapeptide-10 (z. B. Collaxyl von Vincience oder Seriseline von Lipotec) und Hexapeptide-11 (z. B. Peptamide-6 von Arch Personal Care). Ein erfindungsgemäß bevorzugtes Pentadecapeptid ist z. B. der Rohstoff Vinci 01 von Vincience (Pentadecapeptide-1). Ein weiteres bevorzugtes Aminosäureoligomer ist das Peptidderivat L-Glutamylaminoethyl-indol (enthalten in dem Rohstoff Glistin von Exsymol).

Ein erfindungsgemäß besonders bevorzugter Aminosäureoligomer-Wirkstoff ist die Kombination aus N-Palmitoyl-Gly-His-Lys und N-Palmitoyl-Gly-Gln-Pro-Arg, wie sie beispielsweise in dem Rohstoff Matrixyl 3000 von der Firma Sederma erhältlich ist.

Ein weiterer erfindungsgemäß bevorzugter Wirkstoff ist ausgewählt aus Polymeren der Aminosäuren und/oder der N-C₂-C₂₄-Acylaminosäuren.

Erfindungsgemäß kombinierte Polymere der Aminosäuren und/oder der N-C₂-C₂₄-Acylaminosäuren sind bevorzugt ausgewählt aus pflanzlichen und tierischen Proteinhydrolysaten und/oder Proteinen. Tierische Proteinhydrolysate sind z. B. Elastin-, Collagen-, Keratin-, Seiden-, Conchiolin- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Entsprechende Handelsprodukte sind z. B. DiaMin^{®} (Diamalt), Gluadin^{®} (Cognis), Lexein^{®} (Inolex) und Crotein^{®} (Croda). Besonders bevorzugt sind Sojaproteinhydrolysate, besonders bevorzugt Sojaproteinhydrolysate mit einer mittleren Molmasse (Mw) im Bereich von 1200 - 1800 Dalton, bevorzugt im Bereich von 1400 - 1700 Dalton, z. B. unter dem Handelsnamen Ridulisse C^{®} von der Firma Silab erhältlich, und Sojaproteinhydrolysate mit einer mittleren Molmasse (Mw) im Bereich von 600 - 1000 Dalton, bevorzugt 800 Dalton, z. B. unter dem Handelsnamen Phytokine^{®} von Coletica erhältlich. mit Kokosfettsäuren N-acylierten und/oder veresterten Sojaproteinhydrolysaten in Form ihrer Alkalimetallsalze. Kokosfettsäuren umfassen überwiegend Alkancarbonsäuren mit einer Anzahl an Kohlenstoffatomen von 8 - 18, insbesondere Caprylsäure, Caprinsäure, Laurinsäure, Myristinsäure, Palmitinsäure und Stearinsäure. Bevorzugte Alkalimetallsalze sind ausgewählt aus Lithium-, Natrium- und Kaliumsalzen, wobei die Kaliumsalze besonders bevorzugt sind.

Proteinhydrolysate können naturgemäß auch monomere Aminosäuren und Oligopeptide enthalten; ihre Zusammensetzung ist normalerweise nicht definiert.

Ebenfalls bevorzugt ist der Einsatz von Acylderivaten der Proteinhydrolysate, z. B. in Form ihrer Fettsäure-Kondensationsprodukte.

In einer weiteren bevorzugten Ausführungsform sind die erfindungsgemäß kombinierten Polymeren der Aminosäuren ausgewählt aus DNA-Reparaturenzymen.

Erfindungsgemäß bevorzugte DNA-Reparaturenzyme sind Photolyase und T4 Endonuclease V, letztere im Weiteren mit "T4N5" abgekürzt. Diese beiden Enzyme sind im Stand der Technik bereits als so genannte DNA-Reparatur-Enzyme bekannt. Unter DNA-Reparatur ist definitionsgemäß die Spaltung bzw. Entfernung von UV-induzierten Pyrimidindimeren aus der DNA zu verstehen.

Photolyase ist die Kurzbezeichnung für Desoxyribodipyrimidin-Photolyase bzw. DNA-Photolyase, ein Enzym mit der Klassifizierungsnummer EC 4.1.99.3. Eine besonders effiziente Photolyase stammt aus *Anacystis nidulans*, einem phototrophen marinen Mikroorganismus. Die Photolyase aus *A. nidulans* wird in technisch relevanten Mengen mittlerweile aus E. coli gewonnen. Photolyase ist zur Aktivierung auf Licht angewiesen.

Das Enzym T4 Endonuclease V wird vom *den*V-Gen der Bakteriophage T4 produziert und gehört zu den Phosphodiesterasen, die die Nucleinsäuren an der (5'-3')-Bindung hydrolytisch spalten. T4N5 ist auch ohne Lichteinfluss aktiv.

Erfindungsgemäß besonders bevorzugt ist der Einsatz von liposomenverkapselten DNA-Reparaturenzymen. Liposomenverkapselte Photolyase ist im Handel z. B. unter der Produktbezeichnung Photosome™, liposomenverkapselte T4N5 z. B. unter der Bezeichnung Ultrasome™ von der Firma AGI Dermatics, USA, erhältlich.

Erfindungsgemäß besonders bevorzugte Zusammensetzungen sind **dadurch gekennzeichnet, dass** sie zusätzlich zu der Wirkstoffkombination a) und b) als weiteren Inhaltsstoff mindestens eines der Handelsprodukte Photosomes™ oder Ultrasomes™ in Gesamtmengen von 0,1 - 10 Gew.-%, bevorzugt 0,5 - 5,0 Gew.-% und besonders bevorzugt 1,0 - 4,0 Gew.-%, bezogen auf die gesamte erfindungsgemäße Zusammensetzung, enthalten.

Erfindungsgemäß besonders bevorzugte kosmetische oder dermatologische Zusammensetzungen sind **dadurch gekennzeichnet, dass** sie zusätzlich zu der Wirkstoffkombination a) und b) als weiteren Inhaltsstoff mindestens einen kosmetischen Wirkstoff, der ausgewählt ist aus Monomeren, Oligomeren und Polymeren von Aminosäuren, N-C₂-C₂₄-Acylaminosäuren und/oder den Estern und/oder den physiologisch verträglichen Salzen dieser Substanzen, in einer Gesamtmenge von 0,00001 - 2 Gew.-%, bevorzugt 0,0001 - 1 Gew.-%, besonders bevorzugt 0,001 - 0,1 Gew.-% und außerordentlich bevorzugt 0,01 - 0,05 Gew.-%, jeweils bezogen auf den Gehalt an Aktivsubstanz in der gesamten Zusammensetzung, enthalten.

Ein weiterer erfindungsgemäß bevorzugter Wirkstoff ist ausgewählt aus Polysacchariden. Erfindungsgemäß bevorzugte Polysaccharide sind solche, die mindestens einen Desoxyzucker-Baustein, insbesondere Fucose und/oder Rhamnose, enthalten, besonders bevorzugt ausgewählt aus Substanzen mit der INCI-Bezeichnung Biosaccharide Gum-1 (z.B. in dem Handelsprodukt Fucogel^{®} von Solabia), Rhamnosoft^{®} (INCI-Bezeichnung Biosaccharide Gum-2) von Solabia, Fucogenol^{®} (INCI-Bezeichnung Biosaccharide Gum-3) von Solabia und Glycofilm^{®} (INCI-Bezeichnung Biosaccharide Gum-4) von Solabia, weiterhin Mischungen der vorgenannten, mindestens einen Desoxyzucker-Baustein enthaltenden Polysaccharide, beispielsweise der Mischung aus Biosaccharide Gum-2 und Biosaccharide Gum-3, erhältlich als Handelsprodukt Elastinol plus^{®} von Solabia. Weitere erfindungsgemäß bevorzugte Polysaccharide sind ausgewählt aus den Glycosaminoglycanen, besonders bevorzugt Hyaluronsäure, Dextran, Dextransulfat, Chondroitin-4-sulfat und Chondroitin-6-sulfat.

Weitere erfindungsgemäß bevorzugte Polysaccharide sind ausgewählt aus den Glucanen. Bei Glucanen oder Polyglucosanen handelt es sich um eine Klasse von Homopolysacchariden, deren Monomerbaustein ausschließlich Glucose ist. Das Glucose-Molekül kann α-glycosidisch oder βglycosidisch verknüpft, unterschiedlich stark verzweigt oder linear angeordnet sein.

Beispiele für Glucane sind Cellulose, Amylose, Glycogen, Lichenin, Laminarin aus Algen, Pachyman aus Baumpilzen und Hefeglucane mit β-1,3-Bindung; Nigeran, ein aus Pilzen isoliertes Mycodextran (α-1,3-Glucan, α-1,4-Glucan) und Pustulan (β-1,6-Glucan).

Glucane sind unter anderem Bestandteil der Zellwand; sie werden von zahlreichen Mikroorganismen unter bestimmten physiologischen Bedingungen in ihr Milieu abgegeben oder auch erst dort synthetisiert. Dextran, ein primär α-1,6-gebundenes D-Glucan, ist das bedeutendste in dieser Polysaccharid-Gruppe. Weitere technisch interessante und erfindungsgemäß hervorragend geeignete Glucane sind Curdlan (β-1,3-D-Glucan), Pullulan (α-1,4-gebundenes und α-1,6-gebundenes D-Glucan) und Schizophyllan (β-1,3-Grundkette, β-1,6-Seitenkette). Glucane besitzen diverse immunmodulatorische Eigenschaften, auf Zelloberflächen des menschlichen Immunsystems befinden sich entsprechende Glucan-Rezeptoren.

Weitere erfindungsgemäß bevorzugte Polysaccharid-Wirkstoffe sind ausgewählt aus beta-(1,3-1,4)-Glucanen. Besonders bevorzugt sind beta-(1,3-1,4)-Glucane, die zu etwa 70% beta-1,4-Glucosid-Verknüpfungen und zu etwa 30% beta-1,3-Glucosid-Verknüpfungen aufweisen. Derartige beta-(1,3-1,4)-Glucane sind bevorzugt erhältlich aus Extrakten aus Haferkörnern (Avena Sativa (Oat) Kernel Extract).

Weitere erfindungsgemäß bevorzugte Polysaccharid-Wirkstoffe sind ausgewählt aus chemisch modifizierten Cellulose-Derivaten, vorzugsweise Hydroxyalkylcellulosen wie Hydroxyethylcellulose, Hydroxypropylcellulose, Hydroxypropylmethylcellulose, Methylcellulose, Hydroxymethylcellulose, Carboxymethylcellulose, weiterhin insbesondere aus Stärken sowie Stärkeabbauprodukten wie Amylose und Amylopektin, chemisch und/oder thermisch modifizierte Stärken, z. B. Hydroxypropylstärkephosphat, Dihydroxypropyldistärkephosphat oder die Handelsprodukte Dry Flo^{®}, weiterhin Chitosan und dessen Salzen und anderen Derivaten, weiterhin aus Polysacchariden, die Gums oder Gummen bilden, wie beispielsweise Guar-Gum, Xanthan-Gum, Alginate, insbesondere Natriumalginat, Gummi arabicum, Karaya-Gummi, Carrageenane, Johannisbrotkernmehl, Leinsamen-Gums, Schellack und Agar-Agar.

Weitere erfindungsgemäß bevorzugte Wirkstoffe sind ausgewählt aus hyperämisierenden Wirkstoffen.

Erfindungsgemäß bevorzugte kosmetische oder dermatologische Zusammensetzungen sind dadurch gekennzeichnet, dass sie mindestens einen hyperämisierenden Wirkstoff enthalten, der ausgewählt ist aus
- Nicotinsäureestern, insbesondere Nicotinsäurebenzylester (Benzylnicotinat), Nicotinsäurebutoxyethylester (Nicoboxil), Nicotinsäuremethylester, Nicotinsäureethylester, Nicotinsäurehexylester, Nicotinsäureisopropylester, Nicotinsäuremyristylester, Tetrahydrofurfuryl-Nicotinat (Thurfyl-Nicotinat) und Tetrahydrofurfuryl-Nicotinat-Hydrochlorid,
- Pyridin-3-carbaldehyd (β-Pyridincarbaldehyd, Nicotinaldehyd),
- Salicylsäureestern, insbesondere Phenylsalicylat,
- Vanillylethern, insbesondere Vanillylbutylether,
- Carbonsäurevanillylamiden, insbesondere Nonivamid (Nonansäurevanillylamid),
- Scharfstoffen, insbesondere Capsaicin, Cantharidin, Piperin, Gingerol, Zingeron, Myristicin, Safrol, Allicin, Sedamin, Sacculatal, Chalciporon, Isochalciporon, Velleral, Vellerol, und Isothiocyanaten (Senfölen), insbesondere Benzylsenföl,
- Extrakten aus Scharfstoff-Pflanzen, insbesondere aus Capsicum, insbesondere aus Capsicum annuum und Capsicum frutescens, aber auch aus Capsicum chinense, Capsicum baccatum und Capsicum pubescens, weiterhin aus Mauerpfeffer und aus der Zaunrübe,
- Terpentinöl,
- sowie Mischungen hiervon.

Bevorzugt liegen die erfindungsgemäßen kosmetischen oder dermatologischen Zusammensetzungen in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.

In einer besonders bevorzugten Ausführungsform der erfindungsgemäßen Mittel liegen die Mittel als Mikroemulsion vor. Unter Mikroemulsionen werden im Rahmen der Erfindung neben den thermodynamisch stabilen Mikroemulsionen auch die so genannten "PIT"-Emulsionen verstanden. Bei diesen Emulsionen handelt es sich um Systeme mit den 3 Komponenten Wasser, Öl und

Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-Öl-Emulsionen umwandeln. Beim anschließenden Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen.

Ganz besonders bevorzugte erfindungsgemäße Zusammensetzungen sind **dadurch gekennzeichnet, dass** sie in Form einer O/W-Emulsion vorliegen.

In der bevorzugten Ausführungsform als Emulsion enthalten die erfindungsgemäßen Zusammensetzungen mindestens eine oberflächenaktive Substanz als Emulgator oder Dispergiermittel. Geeignete Emulgatoren sind beispielsweise Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsäuren und an C₈-C₁₅-Alkylphenole, C₁₂-C₂₂-Fettsäuremono- und -diester von Anlagerungsprodukten von 1 bis 30 Mol Ethylenoxid an C₃-C₆-Polyole, insbesondere an Glycerin, Ethylenoxid- und Polyglycerin-Anlagerungsprodukte an Methylglucosid-Fettsäureester, Fettsäurealkanolamide und Fettsäureglucamide, C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind, Gemische aus Alkyl-(oligo)-glucosiden und Fettalkoholen, z. B. das im Handel erhältliche Produkt Montanov^{®} 68, Anlagerungsprodukte von 5 bis 60 Mol Ethylenoxid an Rizinusöl und gehärtetes Rizinusöl, Partialester von Polyolen mit 3-6 Kohlenstoffatomen mit gesättigten C₈-C₂₂-Fettsäuren, Sterole (Sterine), insbesondere Cholesterol, Lanosterol, Beta-Sitosterol, Stigmasterol, Campesterol und Ergosterol sowie Mykosterole, Phospholipide, vor allem Glucose-Phospolipide, Fettsäureester von Zuckern und Zuckeralkoholen wie Sorbit, Polyglycerine und Polyglycerinderivate, bevorzugt Polyglyceryl-2-dipolyhydroxystearat (Handelsprodukt Dehymuls^{®} PGPH) und Polyglyceryl-3-diisostearat (Handelsprodukt Lameform^{®} TGI) sowie lineare und verzweigte C₈-C₃₀-Fettsäuren und deren Na-, K-, Ammonium-, Ca-, Mg- und Zn-Salze.

Die erfindungsgemäßen Zusammensetzungen enthalten die Emulgatoren bevorzugt in Mengen von 0,1 bis 25 Gew.-%, besonders bevorzugt 0,5 - 15 Gew.-%, bezogen auf die gesamte Zusammensetzung.

In einer besonders bevorzugten Ausführungsform ist mindestens ein nichtionischer Emulgator mit einem HLB-Wert von 8 und darunter enthalten. Derartige bevorzugte Emulgatoren sind insbesondere Verbindungen der allgemeinen Formel R¹ - O - R², in der R¹ eine primäre lineare Alkyl-, Alkenyl- oder Acylgruppe mit 20 - 30 C-Atomen, bevorzugt ein Behenyl-Rest, und R² Wasserstoff, eine Gruppe mit der Formel -(CₙH₂ₙO)ₓ-H mit x = 1 oder 2 und n = 2 - 4 oder eine Polyhydroxyalkylgruppe mit 4 - 6 C-Atomen und 2 - 5 Hydroxylgruppen, bevorzugt Wasserstoff, darstellt. Eine besonders bevorzugte Verbindung R¹ - O - R² ist Behenylalkohol. Weitere bevorzugt geeignete Emulgatoren mit einem HLB-Wert von 8 und darunter sind die Anlagerungsprodukte von 1 oder 2 Mol Ethylenoxid oder Propylenoxid an Behenylalkohol, Erucylalkohol, Arachidylalkohol oder auch an Behensäure oder Erucasäure. Bevorzugt eignen sich auch die Monoester von C₁₆-C₃₀-Fettsäuren mit Polyolen wie z. B. Pentaerythrit, Trimethylolpropan, Diglycerin, Sorbit, Glucose oder Methylglucose. Beispiele für solche Produkte sind z. B. Sorbitanmonobehenat oder Pentaerythritmonoerucat. Die Anwesenheit von Emulgatoren der allgemeinen Formel R¹ - O - R² kann zu lamellaren Strukturen in der Emulsion führen, die besonders günstige Effekte auf die Wiederherstellung der lamellaren Struktur der Haut haben können. Besonders bevorzugte erfindungsgemäße Zusammensetzungen sind daher **dadurch gekennzeichnet, dass** sie in Form einer lamellare Strukturen aufweisenden Öl-in-Wasser-Emulsion vorliegen.

In einer weiteren besonders bevorzugten Ausführungsform liegen die erfindungsgemäßen Zusammensetzungen in Form einer Wasser-in-Öl-Emulsion vor. Derartige Wasser-in-Öl-Emulsionen können so formuliert werden, dass die eine höhere Viskosität aufweisen, so dass sie sorgfältig in die Haut einzumassieren sind. Dies unterstützt die Anticellulite-Wirkung der erfindungsgemäßen Zusammensetzungen. Genau so gut lassen sich aber auch niedrig-viskose Wasser-in-Öl-Emulsionen formulieren, insbesondere auf Basis des polymeren Wasser-in-Öl-Emulgators PEG-30 Dipolyhydroxystearat, erhältlich z. B. unter dem Handelsnamen Arlacel P 135 von Uniqema. Insbesondere mit Hilfe dieses Emulgators lassen sich sprühbare Wasser-in-Öl-Emulsionen formulieren.

Weitere geeignete Zusatzstoffe sind Verdickungsmittel, z. B. anionische Polymere aus Acrylsäure, Methacrylsäure, Crotonsäure, Maleinsäureanhydrid und 2-Acrylamido-2-methylpropansulfonsäure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel^{®}305, Simulgel^{®} 600, Simulgel^{®} NS und Simulgel^{®} EG der Firma SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol^{®}. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex B.F. Goodrich).

Geeignete nichtionische Polymere sind beispielsweise Polyvinylalkohole, die teilverseift sein können, z. B. die Handelsprodukte Mowiol^{®} sowie Vinylpyrrolidon/Vinylester-Copolymere und Polyvinylpyrrolidone, die z. B. unter dem Warenzeichen Luviskol^{®} (BASF) vertrieben werden.

Weitere bevorzugte Zusatzstoffe sind Lösungsmittel wie Ethanol, Isopropanol, Ethylenglykol, Propylenglykol, Propylenglykolmonoethylether, Glycerin und Diethylenglykol, Parfümöle, Substanzen zur Einstellung des pH-Wertes, Komplexbildner wie EDTA, NTA, β-Alanindiessigsäure und Phosphonsäuren, Treibmittel wie Propan-Butan-Gemische, Pentan, Isopentan, Isobutan, N₂O, Dimethylether, CO₂ und Luft.

Wie bereits weiter oben erwähnt, ist Distelöl ein bevorzugter Inhaltsstoff der erfindungsgemäßen Zusammensetzungen. In erfindungsgemäß bevorzugten Emulsionen enthält die Ölphase der Emulsion, bezogen auf das Gewicht der Ölphase, mindestens 10 Gew.-%, vorzugsweise mindestens 20 Gew.-%, weiter bevorzugt mindestens 30 Gew.-% und insbesondere mindestens 35 Gew.-% Distelöl. Vorzugsweise liegt die Obergrenze der Distelölmenge - bezogen auf das Gewicht der Ölphase - bei 95 Gew.-%, vorzugsweise bei 90 Gew.-% und insbesondere bei 80 Gew.-%.

"Ölphase" der Emulsion sind dabei alle unter Normalbedingungen (20°C, 1013,25 mbar) flüssigen kosmetischen Ölkomponenten.

Nicht-verdickende Stärken oder Stärkederivate können als Emulgatoren die üblichen hydrophilen O/W-Emulgatoren bei der Herstellung stabiler Öl-in-Wasser-Emulsionen ersetzen, wenn sie zusammen mit lipophilen Emulgatoren zum Einsatz kommen. Bevorzugt geeignet für die Zwecke der vorliegenden Erfindung sind thermisch und hydrolytisch, ggf. auch enzymatisch abgebaute Stärken, die sich im kalten Wasser klar lösen. Solche Produkte sind im Handel erhältlich, z.B. unter dem Handelsnamen AEROMYL^{®} (Südstärke). Geeignete Stärke-Derivate sind z.B. oxidativ abgebaute Stärken, z.B. Dialdehydstärken und Dicarboxylstärken und die vernetzten Stärkeester und Stärkeether. Solche, im Handel erhältlichen, weitgehend wasserunlöslichen Produkte sind z.B. das Distärkephosphat, welches z.B. als Mais P04^{®} (Dr. Hauser GmbH.) im Handel ist. Auch quervernetzte Stärkeether z.B. die mit Methylolharnstoff modifizierte "Dimethylimidazolidinone Rice Starch" (gemäß INCI), die unter der Handelsbezeichnung RICE NS (Dr. Hauser GmbH.) erhältlich sind, eignen sich für die Zwecke der Erfindung. Auch hydrophob modifizierte Stärken, z.B. das "Aluminium Starch Octenyl Succinate", eine mit Octenylbern-steinsäureanhydrid modifizierte Stärke, eignet sich für die Zwecke der Erfindung. Die erfindungsgemäß geeigneten modifizierten Stärken sollen feinteilig sein, ihre Teilchengröße sollte unter 1 mm für die löslichen Stärken, und die mittlere Teilchengröße der unlöslichen Stärken sollte unter 20 µ liegen. Durch die erfindungsgemäß geeigneten, nicht-verdickenden modifizierten Stärkeprodukte werden die Öl-in-Wasser-Cremes auch in Abwesenheit von typischen, hydrophilen Öl-in-Wasser-Emulgatoren ausreichend stabilisiert. Es ist daher nicht erforderlich, hydrophile, d.h. wasserlösliche oder ionische Emulgatoren in die Cremes einzusetzen. Bevorzugt enthalten erfindungsgemäße kosmetische Cremes maximal 0,5 Gew.-%, besonders bevorzugt maximal 0,3 Gew.-% und insbesondere 0 bis maximal 0,1 Gew.-% ionische und hydrophile nichtionische Emulgatoren mit HLB-Werten von 6 und darüber.

Zusammenfassend sind erfindungsgemäße Zusammensetzungen bevorzugt, die bezogen auf ihr Gewicht 0,5 bis 5 Gew.-%, vorzugsweise 0,75 bis 4,5 Gew.-%, weiter bevorzugt 1 bis 4 Gew.-% und insbesondere 1,5 bis 3 Gew.-% hydrophob modifizierte Stärke(n) enthalten.

Ein ganz besonders bevorzugtes hydrophob modifiziertes Stärkederivat ist die Aluminium-Octenylsuccinat-Stärke. Bevorzugte erfindungsgemäße Zusammensetzungen enthalten bezogen auf ihr Gewicht 0,5 bis 5 Gew.-%, vorzugsweise 0,75 bis 4,5 Gew.-%, weiter bevorzugt 1 bis 4 Gew.-% und insbesondere 1,5 bis 3 Gew.-% Aluminium-Octenylsucinat-Stärke.

Die erfindungsgemäßen Zusammensetzungen können ferner kosmetische Hilfsstoffe und weitere Wirkstoffe enthalten, wie sie üblicherweise in solchen Zusammensetzungen verwendet werden, z. B. Konservierungsmittel, Konservierungshelfer, Bakterizide, Parfüms, Farbstoffe, Verdickungsmittel, anfeuchtende und/oder feuchthaltende Substanzen, Fette, Öle, Wachse, Alkohole, Polyole, Polymere, Elektrolyte, organische Lösemittel oder Siliconderivate.

Vorteilhafte Konservierungsmittel sind beispielsweise Formaldehydabspalter (wie z. B. DMDM Hydantoin), lodopropylbutylcarbamate, Parabene, Phenoxyethanol, Ethanol, Benzoesäure und dergleichen mehr.

Bevorzugt umfasst das Konservierungssystem ferner auch Konservierungshelfer, wie beispielsweise Octoxyglycerin, 1,2-Pentandiol, 1,5-Pentandiol, 1,2-Hexandiol, 1,6-Hexandiol, 1,2-Heptandiol, 1,2-Octandiol, 1,8-Octandiol, 1,2-Nonandiol, 1,2-Decandiol, 1,10-Decandiol, 1,2-Undecandiol, 1,2-Dodecandiol, 1,2-Tridecandiol oder 1,2-Tetradecandiol.

Weiter vorteilhaft werden der oder die Wirkstoffe gewählt aus der Gruppe, die Catechine und Gallensäureester von Catechinen und wässrige bzw. organische Extrakte aus Pflanzen oder Pflanzenteilen umfasst, die einen Gehalt an Catechinen oder Gallensäureestern von Catechinen aufweisen, wie beispielsweise den Blättern der Pflanzenfamilie Theaceae, insbesondere der Spezies Camellia sinensis (grüner Tee). Insbesondere vorteilhaft sind deren typische Inhaltsstoffe (wie z. B. Polyphenole bzw. Catechine, Coffein, Vitamine, Zucker, Mineralien, Aminosäuren, Lipide).

Catechine stellen eine Gruppe von Verbindungen dar, die als hydrierte Flavone oder Anthocyanidine aufzufassen sind und Derivate des "Catechins" (Catechol, 3,3',4',5,7-Flavanpentaol, 2-(3,4-Dihydroxyphenyl)-chroman-3,5,7-triol) darstellen. Auch Epicatechin ((2R, 3R)-3,3',4',5,7-Flavanpentaol) ist ein vorteilhafter Wirkstoff im Sinne der vorliegenden Erfindung.

Vorteilhaft sind ferner pflanzliche Auszüge mit einem Gehalt an Catechinen, insbesondere Extrakte des grünen Tees, wie z. B. Extrakte aus Blättern der Pflanzen der Spezies Camellia spec., ganz besonders der Teesorten Camellia sinenis, C. assamica, C. taliensis bzw. C. irrawadiensis und Kreuzungen aus diesen mit beispielsweise Camellia japonica. Bevorzugte Wirkstoffe sind ferner Polyphenole bzw. Catechine aus der Gruppe (-)-Catechin, (+)-Catechin, (-)-Catechin-gallat, (-)-Gallocatechingallat, (+)-Epicatechin, (-)-Epicatechin, (-)-Epicatechingallat, (-)-Epigallo-catechin, (-)-Epigallocatechingallat.

In einer weiteren bevorzugten Ausführungsform enthalten die erfindungsgemäßen Zusammensetzungen mindestens ein Flavonoid oder mindestens einen Flavonoid-reichen Pflanzenextrakt.

Die erfindungsgemäß bevorzugten Flavonoide umfassen die Glycoside der Flavone, der Flavanone, der 3-Hydroxyflavone (Flavonole), der Aurone und der Isoflavone. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Dihydroquercetin (Taxifolin), Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxy-ethoxy)-flavon-3-(6-O-(6-deoxy-α-L-mannopyranosyl)-β-D-glucopyranosid)), Diosmin (3',4',7-Trihydroxy-5-methoxyflavanon-7-rhamnoglucosid), Eriodictin und Apigenin-7-glucosid (4',5,7-Trihydroxyflavon-7-glucosid).

Erfindungsgemäß außerordentlich bevorzugte Flavonoide sind α-Glucosylrutin, Naringin und Apigenin-7-glucosid.

Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin, Hesperidinmethylchalkon und Neohesperidindihydrochalkon.

Erfindungsgemäß besonders bevorzugte Zusammensetzungen sind **dadurch gekennzeichnet, dass** sie mindestens ein Flavonoid in einer Gesamtmenge von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung, enthalten.

In einer weiteren bevorzugten Ausführungsform enthalten die erfindungsgemäßen Zusammensetzungen mindestens ein Ubichinon oder ein Ubichinol oder deren Derivate. Ubichinole sind die reduzierte Form der Ubichinone. Die erfindungsgemäß bevorzugten Ubichinone weisen die Formel (UBI-I) auf: 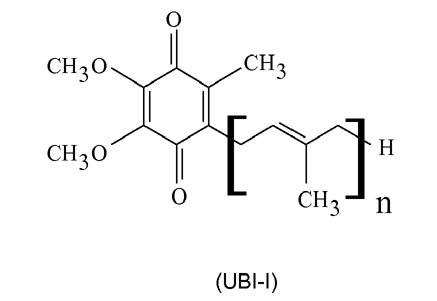 mit n = 6, 7, 8, 9 oder 10.

Besonders bevorzugt ist das Ubichinon der Formel (UBI-I) mit n = 10, auch bekannt als Coenzym Q10.

Erfindungsgemäß besonders bevorzugte Zusammensetzungen sind **dadurch gekennzeichnet, dass** sie mindestens ein Ubichinon, Ubichinol oder ein Derivat hiervon in einer Gesamtmenge von 0,0001 - 1 Gew.-%, bevorzugt 0,001 - 0,5 Gew.-% und besonders bevorzugt 0,005 - 0,1 Gew.-%, jeweils bezogen auf die gesamte Zusammensetzung, enthalten.

In einer weiteren bevorzugten Ausführungsform enthalten die erfindungsgemäßen Zusammensetzungen mindestens ein natürlich vorkommendes Xanthin-Derivat, ausgewählt aus Coffein, Theophyllin, Theobromin und Aminophyllin. Erfindungsgemäß bevorzugt sind die natürlich vorkommenden Xanthin-Derivate in Mengen von 0,0001 bis 1 Gew.-%, besonders bevorzugt 0,001 bis 0,5 Gew.-% und außerordentlich bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen Ectoin. Ectoin ist der Trivialname für 2-Methyl-1,4,5,6-tetrahydropyrimidin-4-carboxylat. Erfindungsgemäß bevorzugt ist Ectoin in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.

In einer weiteren bevorzugten Ausführungsform enthalten die erfindungsgemäßen Zusammensetzungen mindestens eine natürliche Betainverbindung. Erfindungsgemäß bevorzugte natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻), Carnitin (Me₃N⁺-CH₂-CHOH-CH₂-COO⁻), jeweils mit Me = Methyl, und Ergothionein = 2-Mercapto-N^{α}N^{α}N^{α}-trimethyl-L-histidinium-Betain der Formel (BETA-II)

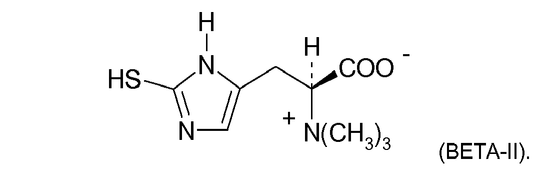

Die Herkunft der eingesetzten natürlichen Betainverbindung kann erfindungsgemäß durchaus synthetisch sein.

Weiterhin können auch Salze und/oder Ester der natürlichen Betainverbindungen erfindungsgemäß bevorzugt eingesetzt werden. Besonders bevorzugte derartige Derivate sind Carnitintartrat, Propionylcarnitin und insbesondere Acetylcarnitin. Beide Enantiomere (D-und L-Form) sind vorteilhaft im Sinne der vorliegenden Erfindung zu verwenden. Es kann auch von Vorteil sein, beliebige Enantiomerengemische, beispielsweise ein Racemat aus D-und L-Form, zu verwenden.

Bevorzugte erfindungsgemäße Zusammensetzungen sind **dadurch gekennzeichnet, dass** sie mindestens eine natürliche Betainverbindung in einer Gesamtmenge von 0,01 bis 5 Gew.-%, bevorzugt 0,1 bis 3 Gew.-%, besonders bevorzugt 0,2 bis 1 Gew.-% und außerordentlich bevorzugt 0,3 - 0,5 Gew.-%, jeweils bezogen auf die gesamte erfindungsgemäße Zusammensetzung, enthalten.

Weitere erfindungsgemäß bevorzugte Zusammensetzungen sind **dadurch gekennzeichnet, dass** sie mindestens einen konditionierenden Wirkstoff enthalten. Unter konditionierenden Wirkstoffen sind erfindungsgemäß solche Substanzen zu verstehen, die auf keratinische Materialien, insbesondere auf die Haut, aufziehen und die physikalischen und sensorischen Eigenschaften sowohl der Haut als auch des Produktes als solchem verbessern. Konditionierungsmittel glätten die oberste Schicht der Haut und machen sie weich und geschmeidig. Über die Auswahl der Konditionierungsmittel (fettig - weniger fettig, schnell oder langsam spreitend, schnell oder langsam in die Haut einziehend und so weiter) lässt sich das Hautgefühl des gesamten Produktes einstellen.

Erfindungsgemäß bevorzugte konditionierende Wirkstoffe sind ausgewählt aus Fettstoffen, insbesondere pflanzlichen Ölen, wie Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls, Lanolin und seinen Derivaten, flüssigen Paraffinölen, Isoparaffinölen und synthetischen Kohlenwasserstoffen, Di-n-alkylethern mit insgesamt 12 bis 36 C-Atomen, z. B. Di-n-octylether und n-Hexyl-n-octylether, Fettsäuren, besonders linearen und/oder verzweigten, gesättigten und/oder ungesättigten C₈₋₃₀-Fettsäuren, Fettalkoholen, besonders gesättigten, ein- oder mehrfach ungesättigten, verzweigten oder unverzweigten Fettalkoholen mit 4 - 30 Kohlenstoffatomen, die mit 1 - 75, bevorzugt 5 - 20 Ethylenoxid-Einheiten ethoxyliert und/oder mit 3 - 30, bevorzugt 9 - 14 Propylenoxid-Einheiten propoxyliert sein können, Esterölen, das heißt Estern von C₆₋₃₀-Fettsäuren mit C₂₋₃₀-Fettalkoholen, Hydroxycarbonsäurealkylestern, Dicarbonsäureestern wie Di-n-butyladipat sowie Diolestern wie Ethylenglykoldioleat oder Propylenglykoldi(2-ethylhexanoat), symmetrischen, unsymmetrischen oder cyclischen Estern der Kohlensäure mit Fettalkoholen, z. B. Glycerincarbonat oder Dicaprylylcarbonat (Cetiol^{®} CC), Mono,- Di- und Trifettsäureestern von gesättigten und/oder ungesättigten linearen und/oder verzweigten Fettsäuren mit Glycerin, die mit 1 - 10, bevorzugt 7 - 9 Ethylenoxid-Einheiten ethoxyliert sein können, z. B. PEG-7 Glyceryl Cocoate, Wachsen, insbesondere Insektenwachsen, Pflanzenwachsen, Fruchtwachsen, Ozokerit, Mikrowachsen, Ceresin, Paraffinwachsen, Triglyceriden gesättigter und gegebenenfalls hydroxylierter C₁₆₋₃₀-Fettsäuren, z. B. gehärteten Triglyceridfetten, Phospholipiden, beispielsweise Sojalecithin, EiLecithin und Kephalinen, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCI-Bezeichnung Dimethicone Copolyol, sowie Polydialkylsiloxanen, die Amin- und/oder Hydroxy-Gruppen enthalten, bevorzugt Substanzen mit den INCI-Bezeichnungen Dimethiconol, Amodimethicone oder Trimethylsilylamodimethicone.

Die Einsatzmenge der Fettstoffe beträgt bevorzugt 0,1 - 99 Gew.-%, besonders bevorzugt 2 - 50 Gew.-% und außerordentlich bevorzugt 5 - 20 Gew.-%, jeweils bezogen auf die gesamte erfindungsgemäße Zusammensetzung.

### Beispiele:

Es wurden Cremes der folgenden Zusammensetzung hergestellt (alle Angaben in Gew.-%):

| | |
|---|---|
| Glycerin 86% pflanzlich | 5 |
| Propandiol-1,2 | 5 |
| Distelöl (Safloröl) raffiniert | 3 |
| Capryl-/Caprinsäure-Triglycerid | 3 |
| Silikonöl 350 cs | 0,5 |
| Cutina MD | 3 |
| Cetearyl Alcohol | 3 |
| Hydrovance | 3 |
| Fucogel 1,5 P | 2 |
| Betafin BP 20 | 2 |
| Simulgel EPG | 1,3 |
| Acnacidol BG | 0,5 |
| Ridulisse C GR | 0,5 |
| Aluminiumstärkeoctenylsuccinat | 3 |
| Phenonip ME | 1 |
| D-Panthenol 75 % | 0,7 |
| d,1-alpha-Bisabolol | 0,5 |
| Antioxidans* | 0,4 |
| Wasser, vollentsalzt | ad 100 |

| | |
|---|---|
| Cutina^{®} MD | Glycerinmono/distearat (INCI-Bezeichnung: Glyceryl Stearate) (Cognis) |
| Fucogel 1,5 P | INCI-Bezeichnung: Aqua, Phenoxyethanol, Biosaccharide Gum-1 (Solabia), Trockensubstanz-Gehalt 0,9 - 1,3 Gew.-% |
| Phenonip^{®} | Aqua, Hydroxybenzoesäuremethylester-Hydroxybenzoesäureethylester-Hydroxybenzoesäurepropylester-Hydroxybenzoesäurebutylester-Phenoxyethanol-Gemisch (ca. 28 % Aktivsubstanz; INCI-Bezeichnung: Phenoxyethanol, Methylparaben, Ethylparaben, Propylparaben, Butylparaben) (Nipa) |
| Betafin^{®} BP 20 | Trimethylglycin (INCI-Bezeichnung: Betain) (Danisco) |
| Hydrovance | Aqua, Bis-N,N-(2-Hydroxyethyl)harnstoff (45 - 55 Gew.-%), Urea, Ammonium lactate (Akzo Nobel) |
| Simulgel EPG | INCI-Bezeichnung: Aqua, Sodium Acrylates/Sodium Acryloyldimethyl taurate Copolymer, Polyisobutene, Caprylyl/Capryl Glucoside (Seppic) |
| Acnacidol BG | Aqua, Butylene Glycol, 10-Hydroxydecanoic Acid, Sebacic Acid, 1,10-Decanediol (40-60 Gew.-% Trockensubstanz) (ISP) |
| Ridulisse C | HYDROLYZED SOY PROTEIN (Silab) |

Eine erfindungsgemäße Creme E enthielt als Antioxidans 0,4 Gew.-% [(4-Hydroxy-3,5-dimethoxy-benzyl]-malonsäure-bis-(2-ethylhexyl)ester. Vergleichscremes V1 bis V4 enthielten als Antioxidans jeweils 0,4 Gew.-% Ethylhexyltriazon (V1, Handelsprodukt: Uvinul T 150), PephaProtect^{®} (V2, Waterlemon fluid extract, preserved with phenoxyethanol, sodium benzoate and potassium sorbate), Herbalia Grape (V3, VITIS VINIFERA (GRAPE SEED EXTRACT), SILICA) und Lipochroman-6 (V4).

Die Cremes wurden in identische Gefäße abgefüllt und unter identischen Bedingungen jeweils 10 Stunden lang mit UV/Vis-Strahlung bestrahlt. Die Ergebnisse zeigt folgende Tabelle:

| | |
|---|---|
| E | keine Farb- und Geruchsveränderung |
| V1 | starke Geruchsveränderung, Produkt riecht ranzig |
| V2 | Farbe leicht verändert, starke Geruchsveränderung, Produkt riecht ranzig |
| V3 | schon vor UV-Bestrahlung durch Eigenfarbe nicht akzeptable, Braunfärbung wird durch Bestrahlung verstärkt. |
| V4 | starke Geruchsveränderung, Produkt riecht ranzig |

Die Ergebnisse zeigen, dass das erfindungsgemäße Mittel E den Vergleichsbeispielen V1 bis V4 deutlich überlegen ist.

Eine besonders bevorzugte erfindungsgemäße Creme besitzt folgende Zusammensetzung:

| | |
|---|---|
| Glycerin 86% pflanzlich | 5 |
| Propandiol-1,2 | 5 |
| Distelöl (Safloröl) raffiniert | 3 |
| Capryl-/Caprinsäure-Triglycerid | 3 |
| Silikonöl 350 cs | 0,5 |
| Cutina MD | 3 |
| Cetearyl Alcohol | 3 |
| Hydrovance | 3 |
| Fucogel 1,5 P | 2 |
| Betafin BP 20 | 2 |
| Simulgel EPG | 1,3 |
| Acnacidol BG | 0,5 |
| Ridulisse C GR | 0,5 |
| Aluminiumstärkeoctenylsuccinat | 3 |
| Phenonip ME | 1 |
| D-Panthenol 75 % | 0,7 |
| d,l-alpha-Bisabolol | 0,5 |
| Vitamin E Acetat | 0,5 |
| Controx KS | 0,1 |
| [(4-Hydroxy-3,5-dimethoxybenzyl]-malonsäure-bis-(2-ethylhexyl)ester | 0,4 |
| Wasser, vollentsalzt | ad 100 |

| | |
|---|---|
| Controx KS: Tocopherol, Hydrogenated Palm Glycerides Citrate (Cognis) | |

## Patentansprüche

1. Kosmetische Zusammensetzung, enthaltend in einem geeigneten Träger
a) mindestens eine Substanz der allgemeinen Struktur (I) 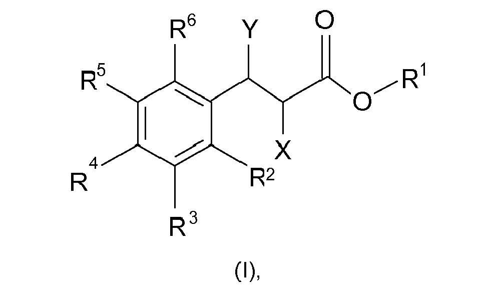 worin
R¹ für ein H-Atom oder eine verzweigte oder lineare C₁-C₃₀-Alkylgruppe oder eine verzweigte oder lineare C₁-C₃₀-Hydroxyalkylgruppe,
R², R⁴ und R⁶ jeweils für ein H-Atom oder eine OH-Gruppe stehen, wobei mindestens einer der Reste R², R⁴ oder R⁶ eine OH-Gruppe ist,
R³ und R⁵ unabhängig voneinander für eine lineare oder verzweigte C₁-C₄-Alkoxygruppe oder eine lineare oder verzweigte C₁-C₆-Alkylgruppe,
X für eine Gruppe -C(=O)OR¹,
und Y für ein H-Atom oder einen gegebenenfalls substituierten Phenylrest Ar stehen,
b) mindestens eine ungesättigte oder aromatische Verbindung, ausgewählt aus natürlichen Öl- oder Fettkomponenten und Duftstoffen, insbesondere Terpenen, die funktionelle Substituenten aufweisen können, gegebenenfalls substituierten Benzylalkoholen und Phenylalkoholen und aldehydischen Duftstoffen.

2. Zusammensetzung gemäß Anspruch 1, **dadurch gekennzeichnet, dass** die natürliche Öl-oder Fettkomponente ausgewählt ist aus Distelöl, wobei bevorzugte Zusammensetzungen, bezogen auf ihr Gewicht, mindestens 2 Gew.-% Distelöl enthalten.

3. Zusammensetzung gemäß Anspruch 1 oder 2, **dadurch gekennzeichnet, dass** der mindestens eine Duftstoff ausgewählt ist aus Terpenen.

4. Zusammensetzung gemäß Anspruch 3, **dadurch gekennzeichnet, dass** das mindestens eine Terpen ausgewählt ist aus Monoterpenen, Sesquiterpenen, Diterpenen, Sesterterpenen, Triterpenen, Tetraterpenen und Polyterpenen.

5. Zusammensetzung gemäß Anspruch 1, 2, 3 oder 4, **dadurch gekennzeichnet, dass** das mindestens eine Terpen ausgewählt ist aus Bisabolol und Limonen.

6. Zusammensetzung gemäß Anspruch 1, 2, 3, 4 oder 5, **dadurch gekennzeichnet, dass** der mindestens eine Duftstoff ausgewählt ist aus alpha-Isomethylionon, Amylcinnamal, Amylcinnamylalkohol, Anisalkohol, Benzylcinnamat, Cinnamal, Cinnamylalkohol, Citral, Citronellol, Cumarin, Limonen, Eugenol, Farnesol, Geraniol, Hexylcinnamal, Hydroxycitronel-Ial, Isoeugenol, Lilial, Linalool, Lyral (Hydroxylsohexyl-3-cyclohexencarboxaldehyd) und Methyl-2-octinoat.

7. Zusammensetzung gemäß Anspruch 1, 2, 3, 4, 5 oder 6, **dadurch gekennzeichnet, dass** die Komponente a) ausgewählt ist aus 4-Methoxy-benzyl-malonsäure-di-2-ethyl-hexylester, 4-Hydroxy-3,5-dimethoxy-benzyl-malonsäure-diethylester, 4-Hydroxy-benzyl-malonsäure-di-2-ethyl-hexylester, 3-Hydroxy-benzyl-malonsäure-di-2-ethyl-hexylester, 2-Hydroxy-benzyl-malonsäure-di-2-ethyl-hexylester, 3,4,5-Trimethoxy-benzyl-malonsäure-di-2-ethyl-hexylester, 2,4,5-Trimethoxy-benzyl-malonsäure-di-2-ethyl-hexylester, 2,3,4-Trimethoxy-benzyl-malonsäure-di-2-ethyl-hexylester, 2,3, 5-Trimethoxy-benzyl-malonsäure-di-2-ethyl-hexylester, 2,3,6-Trimethoxy-benzyl-malonsäure-di-2-ethyl-hexylester, 2,4,6-Trimethoxy-benzyl-malonsäure-di-2-ethyl-hexylester, 2,4-Dimethoxy-benzyl-malonsäure-di-2-ethyl-hexylester, 2,3-Dimethoxy-benzyl-malonsäure-di-2-ethyl-hexylester, 2,5-Dimethoxy-benzyl-malonsäure-di-2-ethyl-hexylester, 3,4-Dimethoxy-benzyl-malonsäure-di-2-ethyl-hexylester, 3, 5-Dimethoxy-benzyl-malonsäure-di-2-ethyl-hexylester, 4-Hydroxy-3-methoxy-benzyl-malonsäure-di-2-ethyl-hexylester, 34,5-Trihydroxy-benzyl-malonsäure-di-2-ethyl-hexylester, 2,4,5-Trihydroxy-benzyl-malonsäure-di-2-ethyl-hexylester, 2,3,4-Trihydroxy-benzyl-malonsäure-di-2-ethyl-hexylester, 2,3,4-Trihydroxy-phenyl-propionsäure-2-ethyl-hexylester, 2,4-Dihydroxy-benzyl-malonsäure-di-2-ethyl-hexylester, 2,3-Dihydroxy-benzyl-malonsäure-di-2-ethyl-hexylester, 2,5-Dihydroxy-benzyl-malonsäure-di-2-ethy-hexytester, 3,4-Dihydroxy-benzyl-malonsäure-di-2-ethyl-hexylester, 3,5-Dihydroxy-benzyl-malonsäure-di-2-ethyl-hexylester, 3-Hydroxy-4-methoxy-benzyl-malonsäure-di-2-ethyl-hexylester, 4-Methoxy-benzyl-malonsäure-diethylester, 3,4,5-Trimethoxy-benzyl-malonsäure-diethylester, 2,4,5-Trimethoxy-benzyl-malonsäure-diethylester, 2,3,4-Trimethoxy-benzyl-matonsäure-diethylester, 2,3,5-Trimethoxy-benzyl-malonsäure-diethylester, 2,3,6-Trimethoxy-benzyl-malonsäure-diethylester, 2,4,6-Trimethoxy-benzyl-malonsäure-diethyester, 2,4-Dimethoxy-benzyl-malonsäure-diethylester, 2,3-Dimethoxy-benzyl-malonsäure-diethylester, 2,5-Dimethoxy-benzyl-malonsäure-diethylester, 3,4-Dimethoxy-benzyl-malonsäure-diethylester, 3,5-Dimethoxy-benzyl-malonsäure-diethylester, 4-Hydroxy-3-methoxy-benzyt-malonsäure-diethylester, 3,4,5-Trihydroxy-benzyl-malonsäure-diethylester, 2,4,5-Trihydroxy-benzyl-malonsäure-diethylester, 2,3,4-Trihydroxy-benzyl-malonsäure-diethylester, 2,4-Dihydroxy-benzyl-malonsäure-diethylester, 2,3-Dihydroxy-benzyl-malonsäure-diethylester, 2,5-Dihydroxy-benzyl-matonsäure-thethylester, 3,4-Dihydroxy-benzyl-malonsäure-diethylester, 3,5-Dihydroxy-benzyl-malonsäure-diethylester, 3,5-Dihydroxy-phenyl-propionsäure-ethylester, 3-Hydroxy-4-methoxy-benzyl-malonsäure-diethylester, 4-Methoxy-benzyl-malonsäure-diphenethylester, 3,4,5-Trimethoxy-benzyl-malonsäure-diphenethylester, 2,4,5-Trimethoxy-benzyl-malonsäure-diphenethylester, 2,3,4-Trimethoxy-benzyl-malonsäure-diphenethylester, 2,3,5-Trimethoxy-benzyl-malonsäure-diphenethylester, 2,3,6-Trimethoxy-benzyl-malonsäure-diphenethylester, 2,4,6-Trimethoxy-benzyl-malonsäure-diphenethylester, 2,4-Dimethoxy-benzyl-malonsäure-diphenethylester, 2,3-Dimethoxy-benzyl-malonsäure-diphenethylester, 2,5-Dimethoxy-benzyl-malonsäure-diphenethylester, 3,4-Dimethoxy-benzyl-malonsäure-diphenethylester, 3,5-Dimethoxy-benzyl-malonsäure-diphenethylester, 4-Hydroxy-3-methoxy-benzyl-malonsäure-diphenethylester, 3,4,5-Trihydroxy-benzyl-malonsäure-diphenethylester, 2,4,5-Trihydroxy-benzyl-malonsäure-diphenethylester, 2,3,4-Trihydroxy-benzyl-malonsäure-diphenethylester, 2,4-Dihydroxy-benzyl-malonsäure-diphenethylester, 2,3-Dihydroxy-benzyl-malonsäure-diphenethylester, 2,5-Dihydroxy-benzyl-malonsäure-diphenethylester, 3,4-Dihydroxy-benzylmalonsäure-diphenethylester, 3,5-Dihydroxy-benzyl-malonsäure-diphenethylester, 3-Hydroxy-4-methoxy-benzyl-malonsäure-diphenethylester, sowie deren Mischungen.

8. Zusammensetzung gemäß Anspruch 1, 2 3, 4, 5, 6 oder 7, **dadurch gekennzeichnet, dass** die Komponente a) ausgewählt ist aus [(4-Hydroxy-3,5-dimethoxybenzyl]-malonsäure-bis-(2-ethylhexyl)ester.

9. Zusammensetzung gemäß Anspruch 1, 2 3, 4, 5, 6, 7 oder 8, **dadurch gekennzeichnet, dass** weiterhin Tocopherol oder ein Tocopherylester enthalten ist.

10. Zusammensetzung gemäß Anspruch 1, 2 3, 4, 5, 6, 7, 8 oder 9, **dadurch gekennzeichnet, dass** Catechine, Proanthocyanidine, Anthocyanidine und Gallotannine in einer Gesamtmenge von 0 - 0,001 Gew.-% enthalten sind.

11. Zusammensetzung gemäß Anspruch 1, 2 3, 4, 5, 6, 7, 8, 9 oder 10, **dadurch gekennzeichnet, dass** sie in Form einer O/W-Emulsion vorliegt.

12. Zusammensetzung gemäß Anspruch 11, **dadurch gekennzeichnet, dass** die Ölphase der Emulsion bezogen auf das Gewicht der Ölphase mindestens 10 Gew.-%, vorzugsweise mindestens 20 Gew.-%, weiter bevorzugt mindestens 30 Gew.-% und insbesondere mindestens 35 Gew.-% Distelöl enthält.

13. Zusammensetzung gemäß Anspruch 1, 2 3, 4, 5, 6, 7, 8, 9, 10, 11 oder 12, **dadurch gekennzeichnet, dass** sie bezogen auf ihr Gewicht 0,5 bis 5 Gew.-%, vorzugsweise 0,75 bis 4,5 Gew.-%, weiter bevorzugt 1 bis 4 Gew.-% und insbesondere 1,5 bis 3 Gew.-% hydrophob modifizierte Stärke(n) enthält.

14. Zusammensetzung nach Anspruch 13, **dadurch gekennzeichnet, dass** sie bezogen auf ihr Gewicht 0,5 bis 5 Gew.-%, vorzugsweise 0,75 bis 4,5 Gew.-%, weiter bevorzugt 1 bis 4 Gew.-% und insbesondere 1,5 bis 3 Gew.-% Aluminium-Octenylsucinat-Stärke enthält.
